(19) Europäisches Patentamt
European Patent Office
Office européen des brevets

(11) EP 1 034 180 B1

# (12) EUROPÄISCHE PATENTSCHRIFT

(45) Veröffentlichungstag und Bekanntmachung des Hinweises auf die Patenterteilung:
**29.03.2006 Patentblatt 2006/13**

(21) Anmeldenummer: **98952612.4**

(22) Anmeldetag: **21.09.1998**

(51) Int Cl.:
***C07H 13/04*** *(2006.01)* ***A61K 31/70*** *(2006.01)*
***C07H 19/04*** *(2006.01)* ***C07H 21/00*** *(2006.01)*

(86) Internationale Anmeldenummer:
**PCT/EP1998/006000**

(87) Internationale Veröffentlichungsnummer:
**WO 1999/015542 (01.04.1999 Gazette 1999/13)**

## (54) LINKER-NUCLEOSID, SEINE HERSTELLUNG UND VERWENDUNG

LINKER NUCLEOSIDE, AND PRODUCTION AND USE OF THE SAME

NUCLEOSIDE LIEUR, MODE DE PREPARATION ET UTILISATION

(84) Benannte Vertragsstaaten:
**AT CH DE FR GB IT LI NL**

(30) Priorität: **22.09.1997 DE 19741738**

(43) Veröffentlichungstag der Anmeldung:
**13.09.2000 Patentblatt 2000/37**

(73) Patentinhaber: **Nanogen Recognomics GmbH**
**65926 Frankfurt am Main (DE)**

(72) Erfinder:
- **MICULKA, Christian**
  **D-65929 Frankfurt am Main (DE)**
- **WINDHAB, Norbert**
  **D-65795 Hattersheim am Main (DE)**
- **BRANDSTETTER, Tilmann**
  **D-65929 Frankfurt am Main (DE)**
- **SCHERER, Stefan**
  **D-64572 Büttelborn (DE)**

(74) Vertreter: **Ackermann, Joachim**
**Postfach 11 13 26**
**60048 Frankfurt am Main (DE)**

(56) Entgegenhaltungen:
**EP-A- 0 057 548** **WO-A-97/28176**
**DE-A- 2 028 906**

- **HAYAKAWA, Y. ET AL.: "O-Allyl protection of guanine and thymine residues in oligodeoxyribonucleotides" J. ORG. CHEM., Bd. 58, Nr. 20, 1993, Seiten 5551-5, XP002094188**
- **DE CLERCQ, E. ET AL.: "Antiviral activity of novel deoxyuridine derivatives" CURR. CHEMOTHER., PROC. INT. CONGR. CHEMOTHER. 10 TH (1978), Bd. 1, Seiten 352-4, XP002094189**
- **ESCHENMOSER, A. ET AL.: "147. Why pentose- and not hexose-nucleic acids?" HELV. CHIM. ACTA, Bd. 76, 1993, Seiten 2161-83, XP002094190**
- **HANNA, M.: "Photochemical cross-linking analysis of protein-nucleic acid interactions in Escherichia coli transcription complexes from lambda Pk promoter" METHODS IN ENZYMOLOGY, Bd. 274, 1996, Seiten 403-418, XP002045925**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 034 180 B1

Printed by Jouve, 75001 PARIS (FR)

## Beschreibung

Die vorliegende Erfindung betrifft ein Linker-Nucleosid, seine Herstellung und Verwendung zur kovalenten Bindung von Biomolekülen an Oligonucleotide, insbesondere p-RNA-Oligonucleotide.

Pyranosyl-Nucleinsäuren (p-NA's) sind im allgemeinen zur natürlichen RNA isomere Strukturtypen, bei denen die Pentose-Einheiten in der Pyranoseform vorliegen und durch Phosphodiestergruppen zwischen den Positionen C-2' und C-4' repetitiv verknüpft sind (Fig. 1). Unter "Nucleobase" werden dabei die kanonischen Nucleobasen A, T, U, C, G, aber auch die Paare Isoguanin/Isocytosin und 2,6-Diaminopurin/Xanthin und im Sinne der vorliegenden Erfindung auch andere Purine und Pyrimidine verstanden. p-NA's, und zwar die von der Ribose abgeleitete p-RNA's, wurden zum erstenmal von Eschenmoser et al. beschrieben (Pitsch, S. et al. Helv. Chim. Acta 1993, 76, 2161; Pitsch, S. et al. Helv. Chim Acta 1995, 78, 1621; Angew. Chem. 1996, 108, 1619-1623). Sie bilden ausschließlich sogenannte Watson-Crickgepaarte. d. h. Purin-Pyrimidin- und Purin-Purin-gepaarte, antiparallele, reversibel "schmelzende", quasi-lineare und stabile Duplices. Homochirale p-RNA-Stränge entgegengesetzten Chiralitätssinns paaren ebenfalls kontrollierbar und sind in der gebildeten Duplex streng nicht-helical. Diese für den Aufbau supramolekularer Einheiten wertvolle Spezifität hängt mit der relativ geringen Flexibilität des Ribopyranosephosphat-Rückgrats sowie mit der starken Neigung der Basenebene zur Strangachse und der hieraus folgenden Tendenz zu intercatenarer Basenstapelung im resultierenden Duplex zusammen und läßt sich letzlich auf die Teilnahme eines 2',4'-cis-disubstituierten Ribopyranoserings am Aufbau des Rückgrates zurückführen. Diese wesentlich besseren Paarungseigenschaften machen p-NA's gegenüber DNA und RNA für die Anwendung des Aufbaus supramolekularer Einheiten zu bevorzugten Paarungssystemen. Sie bilden ein zu natürlichen Nucleinsäuren orthogonales Paarungsystem, d. h. sie paaren nicht mit in der natürlich Form vorkommenden DNA's und RNA's, was im besonderen im diagnostischen Bereich von Bedeutung ist.

Eschenmoser et al. (1993, supra) hat zum ersten Mal eine p-RNA, wie in Fig. 2 dargestellt und nachstehend erläutert, hergestellt.

Hierbei wurde eine geeignete geschützte Nucleobase mit dem Anomerengemisch der Tetrabenzoyl-Ribopyranose durch Einwirken von Bis(trimethylsilyl)acetamid und einer LewisSäure wie z. B. Trimethylsilyl-trifluormethansulfonat zur Reaktion gebracht (analog H. Vorbrüggen. K. Krolikiewicz, B. Bennua, Chem. Ber. 1981. 114, 1234.). Unter Baseneinwirkung (NaOH in THF/Methanol/Wasser im Falle der Purine; gesättigter Ammoniak in MeOH im Falle der Pyrimidine) wurden die Acylschutzgruppen vom Zucker abgespalten, und das Produkt unter saurer Katalyse mit p-Anisaldehyddimethylacetal in 3',4'-Position geschützt. Das Diastereomerengemisch wurde in 2'-Stellung acyliert, das 3', 4'-methoxybenzylidengeschützte 2'-Benzoat durch saure Behandlung, z. B. mit Trifluoressigsäure in Methanol deacetalisiert, und mit Dimethoxytritylchlorid umgesetzt. Die 2'→3'-Wanderung des Benzoats wurde durch Behandlung mit p-Nitrophenol/4-(Dimethylamino)pyridin/Triethylamin/Pyridin/n-Propanol eingeleitet. Fast alle Reaktionen wurden durch Säulenchromatographie aufgearbeitet. Der so synthetisierte Schlüsselbaustein, das 4'-DMT-3'-benzoyl-1'-Nucleobasen-Derivat der Ribopyranose, wurde dann zum Teil phosphityliert bzw. über einen Linker an eine feste Phase gebunden.

Bei der anschließenden automatisierten Oligonucleotidsynthese wurde die trägergebundene Komponente in 4'-Position wiederholt sauer entschützt, ein Phosphoramidit unter Einwirkung eines Kupplungsreagenz, z. B. ein Tetrazolderivat, angekuppelt, noch freie 4'-Sauerstoffatome acetyliert und das Phosphoratom oxidiert, um so das oligomere Produkt zu erhalten. Anschließend wurden die restlichen Schutzgruppen abgespalten, das Produkt über HPLC gereinigt und entsalzt.

Nachteil des bereits bekannten p-RNA Oligonucleotide ist, daß keine Methoden bekannt sind, andere Biomoleküle an diese Oligonucleotide kovalent zu binden.

Aufgabe der vorliegenden Erfindung war es daher, geeignete Konstrukte bereitzustellen, die eine kovalente Bindung anderer Biomoleküle an Oligonucleotide, insbesondere an p-RNA-Oligonucleotide ermöglichen.

Ein Gegenstand der vorliegenden Erfindung ist daher ein Linker-Nucleosid der Formel (I) gemäß Anspruch I.

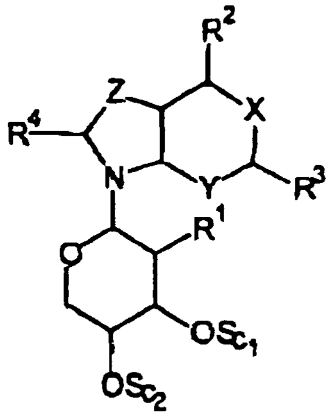

(I)

worin
$R^1$ gleich H, OH, Hal mit Hal gleich Br oder Cl,
$R^2$, $R^3$ und $R^4$ unabhängig voneinander, gleich oder verschieden, jeweils H oder $(C_nH_{3n}NR^{10}R^{11}$ mit $R^{10}R^{11}$ verbunden aber einen Rest der Formel

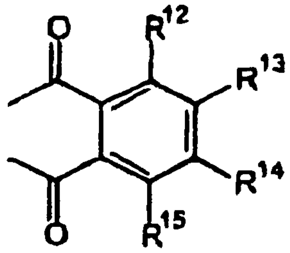

(V),

worin $R^{12}$, $R^{13}$, $R^{14}$ und $R^{15}$ unabhängig voneinander, gleich oder verschieden, jeweils H, $C_4H2_{n-1}$ oder $C_nH_{2n-1}$, mit n gleich eine ganze Zahl von I-12, vorzugsweise 1-8, insbesondere 1-4, oder $OR^7$, wobei $R^7$ gleich H. $C_nH_{2n+1}$ oder $C_nH_{2n-1}$, wobei n die oben genannte Bedeutung hat, $-C(O)R^8$ mit $R^8$ gleich ein linearer oder verzweigter, gegebenenfalls substituierter Alkyl-, Aryl-, vorzugsweise Phenyl-Rest,
X, Y und Z unabhängig voneinander, gleich oder verschieden, jeweils =N-, $=C(R^9)$- oder $-N(R^{9-})$- mit $R^9$ und $R^{9-}$ unabhängig voneinander, gleich oder verschieden, jeweils H oder $C_nH_{2n-1}$ oder $(C_nH_{2n})NR^{10}R^{11}$ mit den oben genannten Bedeutungen, bedeutet, und
$S_{c1}$, und $S_{c2}$ unabhängig voneinander, gleich oder verschieden, jeweils H oder eine Schutzgruppe ausgewählt aus einer Acyl-, Trityl- oder Allyloxycarbonylgruppe, vorzugsweise eine Benzoyl- oder 4, 4'-Dimethoxytrityl-(DMT-)gruppe, oder ein Phosphoester(III), Phosphoester(V), Thiophosphat(V), Phosphonat oder Phosphoramidit,
oder der Formel (III) gemäß Anspruch 1.

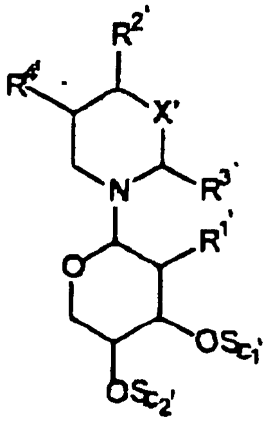

(III)

worin $R^{1'}$ gleich H, OH oder Hal mit Hal gleich Br oder Cl.
$R^{2'}$,$R^{3-}$ und $R^{4-}$ unabhängig voneinander, gleich oder verschieden, jeweils H oder
$(C_nH_{2n})NR^{10}R^{11'}$, wobei $R^{10'}$, $R^{11-}$, unabhängig voneinander die oben genannte Bedeutung von $R^{10}$ bzw. $R^{11}$ hal, und
X' jeweils =N-, =C($R^9$)- oder - N($R^{9-}$)- bedeutet, wobei $R^{9-}$ und $R^{9-}$ unabhängig voneinander die oben genannte Bedeutung von $R^9$ bzw. $R^{9'}$ haben, und
$S_{c1'}$ bzw. $S_{c2'}$ die oben genannte Bedeutung von $S_{c1}$ bzw. $S_{c2}$ haben.

Die erfindungsgemäße Pentose ist im allgemeinen ein Ribose. Arabinose. Lyxose und/oder Xylose, vorzugsweise eine Ribopyranose, wobei der Pentopyranosyl-Teil D-konfiguriert, aber auch L-konfiguriert sein kann.

Der Pentopyranosyl-Nucleosidrest der Linker-Nucleoside nach Formel (I) bzw. (III) kann auch Pentofuranosyl-purin, -2,6-diaminopurin, -6-purinthiol, -pyridin, -pyrimidin,--adenosin, -guanosin, -isoguanosin, -6-thioguanosin, -xanthin, -hypoxanthin, -thymidin,--cytosin, -isocytosin , -indol, -tryptamin, N-phthaloyltryptamin, -uracil, -coffein, -theobromin, -theophyllin, -benzotriatol, insbesondere ein Pentopyranosyl-purin, -pyrimidin, -adenosin, -guanosin, -thymidin, -cytosin, tryptamin, -N-phthalotryptamin oder -uracil sein.

Die erfindungsgemäßen Linker-Nucleoside sind folglich Verbindungen mit funktionellen Gruppert, die kovalent Biomoleküle, an z. B. modifizierte Nucleinsäuren, wie p-NA's, vorzugsweise pRNA's, binden können. Für p-NA's ist dies besonders vorteilhaft, da hier noch keine Linker bekannt sind.

Beispielsweise fallen hierunter Pentopyranosyl-Nucleoside, bei denen $R^2$, $R^3$, $R^4$, $R^{2-}$, $R^{3-}$ und/oder $R^{4-}$ ein 2-Phthalimidoethyl- oder Allyloxy-Rest bedeutet. Bevorzugt sind gemäß der vorliegenden Erfindung beispielsweise Uracil-basierende Linker, bei denen vorzugsweise die 5-Position des Uracils modifiziert wurde, z. B. N-Phthaloylaminoethyluracil, aber auch Indolbasierende Linker, vorzugsweise Tryptaminderivate, wie z. B. N-Phthaloyltryptamin.

In einer besonderen Ausführungsform wird beispielsweise ein Linker gemäß Formel (III) oder (IV), worin $R^{4-}$ $(C_nH_{2n})NR^{10-}R^{11-}$ bedeutet und $R^{10-}R^{11-}$ über einen Rest der Formel (V) mit der bereits bezeichneten Bedeutung verbunden ist, durch folgendes Verfahren auf vorteilhafte Weise hergestellt:

(a) eine Verbindung der Formel (III) mit $R^{4'}$ gleich $(C_nH_{2n})OS_c]$ oder $(C_nH_{2n})$Hal, worin n die oben genannte Bedeutung hat, $S_{c1}$ eine Schutzgruppe, vorzugsweise eine Mesylal-Gruppe, und Hal Chlor oder Brom bedeutet, wird mit einem Azid, vorzugsweise in DMF, umgesetzt, anschließend wird
(b) das Reaktionprodukt aus (a), vorzugsweise mit Triphenylphosphin z. B. in Pyridin reduziert, dann
(c) das Reaktionsprodukt aus (b) mit einem entsprechenden Phthalimid, z. B. N-Ethoxycarbonylphthalimid umgesetzt, und
(d) das Reaktionsprodukt aus (c) mit einer entsprechenden geschützten Pentose, z. B. Ribosetetrabenzoat, umgesetzt, und schließlich
(e) werden gegebenenfalls die Schutzgruppen, z. B. mit Methylat, abgespalten, und dann gegebenenfalls in einen für die Oligonucleotidsynthese geeigneten phosphorylierten Baustein überführt.

Daneben weisen Indolderivate als Linker den Vorzug der Fluoreszenzfähigkeit auf und sind daher für Nanotechnologie-Anwendungen, bei denen es ggf. um den Nachweis kleinster Substanzmengen geht, besonders bevorzugt. So wurden Indol-1-riboside bei N. N. Suvorov et al., Biol. Ahtivn. Soedin., Akad. Nauk SSSR 1965, 60 und Tetrahedron 1967, 23, 4653 bereits beschrieben. Allerdings gibt es kein analoges Verfahren, 3-substituierte Derivate herzustellen..

Im allgemeinen erfolgt ihre Herstellung über die Bildung eines Aminals der ungeschützten Zuckerkomponente und einem Indolin, welches dann durch Oxidation in das Indol-1-ribosid übergeführt wird. Beschrieben wurden z. B. Indol-1-glucoside und -1-arabinoside (Y. V. Dobriynin et al, Khim.-Farm. Zb. 1979, 12, 33), deren 3-substituierte Derivate meist über Vielsmeier-Reaktion hergestellt wurden. Dieser Weg der Einführung von Aminoethyl-Einheiten in 3-Position des Indols ist für eine industrielle Anwendung jedoch zu aufwendig.

In einer weiteren besonderen Ausführungsform wird daher beispielsweise ein Linker gemäß Formel (I), worin X und Y unabhängig voneinander, gleich oder verschieden, jeweils =C($R^{16}$) mit $K^{16}$ gleich H oder $C_nH_{2n}$ und Z =C($R^{16}$)- mit $R^{16}$ gleich ($C_nH_{2n}$)$NR^{10}R^{11}$ durch folgendes Verfahren auf vorteilhafte Weise hergestellt:

(a) das entsprechende Indolin, z. B. N-Phthaloyltryptamin, wird mit einer Pentose, z. B. D-Ribopyranose, zum Nucleosidtriol umgesetzt, dann werden
(b) die Hydroxylgruppen des Pentose-Teils des Produktes aus (a) vorzugsweise mit Acylgruppen, z. B. mittels Essigsäureanhydrid, geschützt, anschließend wird
(c) das Produkt aus (b), z. B. durch 2,3-Dichlor-5,6-dicyanoparachinon, oxidiert, und
(d) gegebenenfalls werden die Hydroxyl-Schutzgruppen des Pentose-Teils des Produktes aus
(e) z. B. mittels Methylat abgespalten, und dann gegebenenfalls in einen für die Oligonucleotidsynthese geeigneten phosphorylierten Baustein überführt.

Die beschriebenen Verfahren lassen sich jedoch nicht nur bei Ribopyranosen anwenden, sondem auch bei Ribofuranosen und 2'-Deoxyribofuranosen bzw. 2'-Deoxyribopyranosen, was besonders vorteilhaft ist. Als Nucleosidierungspartner der Zucker wird vorzugsweise Tryptamin. insbesondere N-Acylderivate des Tryptamins, vor allem N-Phthaloyltryptamin verwendet. Die restlichen Linker-Nucleoside lassen sich in analoger oder dem Fachmann bekannten Weise herstellen.

In einer weiteren Ausführungsform werden die 4'- geschützten, vorzugsweise, die 3', 4'-geschützten Linker-Nucleoside in einem weiteren Schritt phosphityliert oder an eine feste Phase gebunden.

Die Phosphitylierung erfolgt beispielsweise durch Phosphorigsäuremonoallylesterdiisopropyl-amidchlorid in Anwesenheit einer Base, z. B. N-Ethyldiisopropylamin oder durch Phosphortrichlorid und Imidazol bzw. Tetrazol und nachfolgender Hydrolyse unter Basenzusatz. Im ersten Fall ist das Produkt ein Phosphoramidit und im zweiten Fall ein H-Phosphonat. Die Bindung eines geschützten erfindungsgemäßen Pentopyranosyl-Nucleosids an eine feste Phase, z. B. "long-chain-alkylamino-controlled pore glass" (CPG, Sigma Chemie, München) kann beispielsweise wie bei Eschenmoser et al. (1993) beschrieben erfolgen.

Die erhaltenen Verbindungen dienen z. B. für die Herstellung von Pentopyranosyl-Nucleinsäuren, die einen der erfindungsgemäßen Linker enthalten.

Ein weiterer Gegenstand der vorliegenden Erfindung ist daher ein Verfahren zur Herstellung einer Nucleinsäure, mit folgenden Schritten:

(a) in einem ersten Schritt wird ein geschütztes Nucleosid oder ein geschütztes Linker-Nucleosid an eine feste Phase gebunden wird und
(b) in einem zweiten Schritt wird das gemäß Schritt (a) an eine feste Phase gebundene 3'-, 4'-geschützte Nukleosid um ein phosphityliertes 3'-, 4'-geschütztes Nucleosid bzw. Linker-Nucleosid verlängert, anschließend z. B. durch eine wäßrige Jodlösung oxidiert, und
(c) Schritt (b) solange mit gleichen oder unterschiedlichen Nucleosiden bzw. Linker-Nucleosiden wiederholt, bis die gewünschte Nucleinsäure vorliegt, wobei die Nucleinsäure mindestens ein erfinderisches Linker-Nucleosid enthält.

Als Kupplungsreagenz bei Einsatz von Phosphoramiditen eignen sich saure Aktivatoren wie Pyridinium-Hydrochlorid, besonders Benzimidazoliumtriflat, vorzugsweise nach Umkristallisieren in Acetonitril und nach Lösen in Acetonitril, da im Gegensatz zu 5-(4-Nitrophenyl)-1H-tetrazol als Kupplungstreagenz keine Verstopfung der Kupplungsreagenz-Leitungen und eine Verunrseinigung des Produktes erfolgt.

Als Kupplungsreagenz beim Einsatz von H-Phosphonaten eignen sich besonders Arylsulfonylchloride, Diphenylchlorophosphat, Pivaloylchlorid oder Adamantoylchlorid.

Weiterhin ist es vorteilhaft durch Zusatz von einem Salz, wie Natriumchlorid, zur schutzgruppenabspaltenden Hydrazinolyse von Oligonukleotiden, insbesondere von p-NA's, vorzugsweise von p-RNA's, Pyrimidinbasen, vor allem Uracil und Thymin, vor einer Ringöffnung zu schützen, die das Oligonukleotid zerstören wurde. Allyoxygruppen können vorzugsweise durch Palladium [Pd(O)]-Komplexe z.B. vor der Hydrazinolyse abgespalten werden.

In einer weiteren besonderen Ausfuhrangsform können in Schritt (a) und/oder Schritt (b) auch Pentofuranosyl-nucleoside, z. B. das in ihrer natürlichen Form vorkommende Adenosin, Guanosin, Cytidin. Thymidin und/oder Uracil, neben Pentopyranosyl-nuclensiden eingebaut werden, was z. B. zu einer gemischten p-NA-DNA bzw. p-NA-RNA führt.

In einer anderen besonderen Ausruhrungsform kann in einem weiteren Schritt ein Allyloxy-Linker der formel

$S_{c4}NH(C_0H_{2n})CH(OPS_{c5}S_{c6})C_nH_{2n}S_{c7}$ (VI)

worin $S_{c4}$ und $S_{c7}$ unabhängig voneinander, gleich oder verschieden, jeweils eine Schutzgruppe insbesondere ausgewählt aus Fmoc und/oder DMT,
$S_{c5}$ und $S_{c6}$ unabhängig voneinander, gleich oder verschieden, jeweils eine Allyloxy- und/oder Diisopropylamino-Gruppe bedeuten, eingebaut werden, n hat die bereits oben genannte Bedeutung.

Ausgehend von z. B. Lysin können somit in wenigen Reaktionsschritten amino-terminale Linker aufgebaut werden, die sowohl eine aktivierbare Phosphorverbindung als auch eine säurelabile Schutzgruppe, wie DMT, tragen und daher leicht in der automatisierbaren Oligonucleotidsynthese verwendet werden können (siehe z. B. P. S. Nelson et al., Nucleic Acid Res. 1989, 17, 7179; L. J. Arnold et al., WO 8902439). Das Repertoire wurde in der vorliegenden Erfindung durch einen Lysin-basierender Linker erweitert, bei dem anstelle der sonst üblichen Cyanoethyl-Gruppe am Phosphoratom eine Allyloxy-Gruppe eingebracht wurde, und welcher daher in vorteilhafter Weise in der Noyori-Oligonucleotid-Methode eingesetzt werden kann (R. Noyori, J. Am. Chem. Soc. 1990, 112, 1691-6).

Die vorliegende Erfindung erstreckt sich daher auch auf Linker-Nucleoside enthaltend einen Allyloxy-Linker der Formel

$S_{c4}NH(C_nH_{2n})CH(OPS_{c5}S_{c6})C_nH_{2n}S_{c7}$ (VI)

worin $S_{c4}$ und $S_{c7}$ unabhängig voneinander, gleich oder verschieden, jeweils eine Schutzgruppe insbesondere ausgewählt aus Fmoc und/oder DMT,
$S_{c5}$ und $S_{c6}$ unabhängig voneinander, gleich oder verschieden, jeweils eine Allyloxy- und/oder Diisopropylamino-Gruppe und n wie oben bezeichnet, bedeuten.

Ein besonders bevorzugter Allyloxy-Linker ist (2-(S)-N-Fmoc-O'-DMT-$O^{2-}$allyloxydiisopropylaminophosphinyl-6-amino-1,2-hexandiol).

Ein weiterer Gegenstand der vorliegenden Erfindung ist daher auch eine Nucleinsäure, die mindestens ein erfindungsgemäßes Linker-Nucleosid und gegebenenfalls mindestens einen erfindungsgemäßen Allyloxy-Linker enthält. Besonders bevorzugt ist eine Pentopyranosyl-Nucleinsaure, da p-NA's und insbesondere die p-RNA's untereinander stabile Duplices bilden und im allgemeinen nicht mit den in ihrer natürlichen Form vorkommenden DNA's und RNA's paaren. Diese Eigenschaft macht p-NA's zu bevorzugten Paarungssystemen.

Solche Paarungssysteme sind supramolekulare Systeme nicht kovalenter Wechselwirkung, die sich durch Selektivität, Stabilität und Reversiblität auszeichnen, und deren Eigenschaften bevorzugt thermodynamisch, d. h. durch Temperatur, pH-Wert und Konzentration beeinflußt werden. Solche Paarungssysteme können z. B. aufgrund ihrer selektiven Eigenschaften auch als "molekularer Klebstoff" für die Zusammenführung von unterschiedlichen Metallclustern zu Cluster-Verbänden mit potentiell neuen Eigenschaften verwendet werden [siehe z. B. R. L. Letsinger, et al., Nature 1996, 382, 607-9; P. G. Schultz et al., Nature 1996, 382, 609-11]. Folglich eignen sich die p-NA's auch für die Anwendung im Bereich der Nanotechnologie, beispielsweise zur Herstellung neuer Materialien, Diagnostika und Therapeutika sowie mikroelektronischer, photonischer bzw. optoelektronischer Bauteile und für das kontrollierte Zusammenführen molekularer Species zu supramolekularen Einheiten, wie z. B. für den (kombinatorischen) Aufbau von Proteinassemblies [siehe z. B. A. Lombardi, J. W. Bryson. W. F. DeGrado. Biopolymers (Pept. Sci.) 1997, 40, 495-504], da p-NA's Paarungssysteme bilden, die stark und thermodynamisch kontrollierbar sind. Eine weitere Anwendung ergibt sich daher gerade im diagnostischen und drug discovery-Bereich durch die Möglichkeit, funktionelle, bevorzugt biologische Einheiten wie Proteine oder DNA/RNA-Abschnitte, mit einem p-NA-Code zu versehen, der nicht mit den natürlichen Nucleinsäuren interferiert (siehe z. B. WO93/20242).

Ein weiterer Gegenstand der vorliegenden Erfindung ist ein Konjugat enthaltend ein erfindungsgemäßes Linker-Nucleosid bzw. eine erfinderische Nucleinsäure und ein Biomolekül.

Unter Biomolekül versteht man gemäß der vorliegenden Erfindung z. B. ein Peptid oder Protein, wie z. B. einen Rezeptor, einen Antikörper oder funktionelle Teile davon oder ein Enzym, sowie eine Nucleinsäure wie DNA oder RNA, oder Zellbestandteile wie Lipide, Glykoproteine, Filamentbestandteile, oder Viren, Virenbestandteile wie Kapside, Viroide, und deren Derivate wie z. B. Acetate. Funktionelle Teile von Antikörper sind beispielsweise Fv-Fragmente (Skerra & Plückthun (1988) Science 240, 1038), einzelkettige Fv-Fragmente (scFv; Bird et al (1988), Science 242, 423; Huston et al. (1988) Proc. Natl. Acad. Sci. U.S.A., 85, 5879) oder Fab-Fragmente (Better et al. (1988) Science 240, 1041).

Die erfindungsgemäßen Konjugate aus Effektormolekülen und bevorzugt peptidischen, aber im Gegensatz zu PNA selektiven und kontrollierbaren Paarungssystemen sind dann vorteilhaft, wenn reversibel supramolekulare *assemblies* aufgebaut werden sollen, deren Wirkung, wie z. B. Bindung, Inhibition, Auslösung eines physiologischen Effektes, sich von der Wirkung der einzelnen Effektormoleküle unterscheidet.

Ein Ansatz, peptidische "Klebe"-Einheiten zur Bildung von homo- oder heterodimeren *assemblies* zu verwenden, wird z. B. in WO 94/28173 beschrieben:

Assoziierungspeptide (Hexa- oder Heptapeptide) einer festeelegten Sequenz führen Effektoreinheiten, wie z. B. Proteine, zu supramolekularen Einheiten zusammen. Höhere Flexibilität kann eine solchen Methode durch Steuerbarkeit dieses Assoziierungsvorganges erhalten, die im allgemeinen mit den Assoziierungspeptiden nicht realisiert werden kann, in vorteilhafter Weise jedoch mit den Paarungssystemen der vorliegenden Erfindung.

So beschreibt z. B. WO 96/13613 eine Methode zum Auffinden einer Substanz, die eine biologische Wirkung durch die Multimerisierung von Proteinen auslöst, indem man zuerst eine Substanz 1 durch einen Test ermittelt, die an ein Protein bindet, dann eine zweite Substanz II ermittelt, die an ein zweites Protein bindet und dann die beiden Substanzen 1 und II kovalent über einen Linker verbindet, so daß eine Dimerisierung der beiden Proteine ausgelöst wird. Diese Dimerisierung führt dann den gewünschten biologischen Effekt herbei. Größere Flexibilität kann eine solche Methode erhalten, wenn das Verbinden der beiden Substanzen I und II nicht kovalent, sondern durch ein Paarungssystem wie das erfindungsgemäße Oligomer oder Konjugat erfolgt. Durch die Steuerbarkeit dieser Paarung, beispielsweise durch Temperatur oder pH. kann der Dimerisierungsvorgang der Proteine beobachtet oder dessen Ausmaß gesteuert werden. Die erfindungsgemäßen Paarungssysteme haben z. B. gegenüber den Systemen aus WO 96/13522 den Vorteil, daß sie Nuclease-stabil sind.

Ein Biomolekül, z. B. DNA oder RNA, kann zum nicht-kovalenten Verbinden (Linken) mit einem anderen Biomolekül, z. B. DNA oder RNA, verwendet werden, wenn beide Biomoleküle Abschnitte enthalten, die aufgrund komplementärer Sequenzen von Nucleobasen durch Ausbildung von Wasserstoffbrücken aneinander binden können. Derartige Biomoleküle finden z. B. in analytischen Systemen zur Signalamplifizierung Verwendung, wo ein in seiner Sequenz zu analysierendes DNA-Molekül über einen solchen nicht-kovalenten DNA-Linker zum einen an einen festen Träger immobilisiert, und zum anderen an ein signalverstärkendes branchedDNA-Molekül (bDNA) gebunden werden soll (siehe z. B. S. Urdea, Bio/Technol. 1994, 12, 926 oder US-Patent Nr. 5,624,802). Ein wesentlicher Nachteil der zuletzt beschriebenen Systeme ist, daß sie den Verfahren zur Nucleinsäure-Diagnostik durch Polymerase-Chain-Reaction (PCR) (K. Mullis, Methods Enzymol. 1987, 155, 335) hinsichtlich der Empfindlichkeit bis jetzt unterlegen sind. Das ist u. a. darauf zurückzuführen, daß die nicht-kovalente Bindung vom festen Träger an das zu analysierende DNA-Molekül ebenso wie die nicht-kovalente Bindung des zu analysierenden DNA-Moleküls nicht immer spezifisch erfolgt, wodurch es zu einer Vermischung der Funktionen "Sequenzerkennung" und "nicht-kovalente Bindung" kommt. Die Verwendung von p-NA's als orthogonales Paarungssystem, welches nicht in das DNA- bzw. RNA-Paarungsgeschehen eingreift, löst dieses Problem auf vorteilhafte Weise, wodurch die Empfindlichkeit der beschriebenen analytischen Verfahren deutlich erhöht werden kann.

In einer bevorzugten Ausführungsform handelt es sich dabei um p-RNA/DNA- bzw. p-RNA/RNA-Konjugate.

Konjugate werden dann vorzugsweise verwendet, wenn die Funktionen "Sequenzerkennung" und .,nicht-kovalente Bindung" in einem Molekül realisiert werden müssen, da die erfindungsgemäßen Konjugate zwei zueinander orthogonale Paarungssysteme enthalten.

Für die Herstellung von Konjugaten sind sowohl sequentielle als auch konvergente Verfahren geeignet.

In einem sequentiellen Verfahren wird z. B. nach erfolgter automatisierter Synthese eines p-RNA-Oligomeren direkt am gleichen Synthesizer - nach Umstellung der Reagenzien und des Kupplungsprotokolls - z. B. ein DNA-Oligonukleotid weitersynthetisiert. Dieser Vorgang läßt sich auch in umgekehrter Reihenfolge durchführen.

In einem konvergenten Verfahren werden z. B. p-RNA-Oligomere mit Aminoterminalen-Linkem und z. B. DNA-Oligomere mit z. B. Thiol-Linkern in getrennten Vorgängen synthetisiert. Anschließend erfolgt vorzugsweise eine Jodacetylierung des p-RNA-Oligomeren und die Kupplung der beiden Einheiten nach literaturbekannten Protokollen (T. Zhu et al., Bioconjug. Chem. 1994, 5, 312).

Konvergente Verfahren erweisen sich aufgrund ihrer Flexibilität als besonders bevorzugt.

Unter dem Begriff Konjugat im Sinne der vorliegenden Erfindung sind auch sogenannte Arrays zu verstehen. Arrays sind Anordnungen von immobilisierten Erkennungsspecies, die speziell in der Analytik und Diagnostik eine wichtige Rolle bei der simultanen Bestimmung von Analyten spielen. Beispiele sind Peptide-Arrays (Fodor et al., Nature 1993, 364, 555) und Nucleinsäure-Arrays (Southem et al. Genomics 1992, 13, 1008; Heller, US-Patent Nr. 5,632,957). Eine höhere Flexibilität dieser Arrays kann dadurch erreicht werden, daß die Erkennungsspecies an codierende Oligonucleotide gebunden werden und die zugehörigen, komplementären Stränge an bestimmte Positionen auf einem festen Träger. Durch Aufbringen der codierten Erkennungsspecies auf den "anti-codierten" festen Träger und Einstellung von Hybridisierungsbedingungen werden die Erkennungsspecies an den gewünschten Positionen nicht-kovalent gebunden. Dadurch können verschiedene Typen von Erkennungsspecies, wie z. B. DNA-Abschnitte, Antikörper, nur durch Anwendung von Hybridisierungsbedingungen gleichzeitig auf einem festen Träger angeordnet werden (siehe Fig. 4.). Voraussetzung hierzu sind aber äußerst starke, selektive - um die codierenden Abschnitte möglichst kurz zu halten - und mit natürlicher Nucleinsäure nicht interferierender Codons und Anticodons notwendig, p-NA's, vorzugsweise p-RNA's eignen sich hierzu in besonders vorteilhafter Weise.

Unter dem Begriff "Träger" versteht man im Sinne der vorliegenden Erfindung Material, insbesondere Chipmaterial, das in fester oder auch gelartiger Form vorliegt. Als Trägermaterialien eignen sich beispielsweise Keramik, Metall, insbesondere Edelmetall, Gläser, Kunststoffe, kristalline Materialien bzw. dünne Schichten des Trägers, insbesondere der genannten Materialien, oder (bio)molekulare Filamente wie Cellulose, Gerüstproteine.

Ein anderer Gegenstand der vorliegenden Erfindung ist daher ein Träger, an den mindestens ein erfindungsgemäßes Linker-Nucleosid, mindestens eine erfindungsgemäße Nucleinsäure und/oder mindestens ein erfindungsgemäßes Konjugat immobilisiert ist.

Unter dem Begriff "immobilisiert" versteht man im Sinne der vorliegenden Erfindung die Ausbildung einer kovalenten Bindung, quasi-kovalenten Bindung oder supramolekularen Bindung durch Assoziation von zwei oder mehreren molekularen Spezien wie linear konstituierte Moleküle, insbesondere Peptide, Peptoide, Proteine, lineare Oligo- oder Polysaccharide, Nukleinsäuren und deren Analoga, oder Monomere wie Heterocyclen, insbesondere Stickstoffheterocyclen, oder nichtlinear konstituierte Moleküle wie verzweigte Oligo- oder Polysaccharide oder Antikörper und deren funktionelle Teile wie Fv-Fragmente, einzelkettige Fv-Fragmente (scFv) oder Fab-Fragmente.

Als Trägermaterialien eigenen sich beispielsweise Keramik, Metall, insbesondere Edelmetall, Gläser, Kunststoffe, kristalline Materialien bzw. dünne Schichten des Trägers, insbesondere der genannten Materialien, oder (bio) molekulare Filamente wie Cellulose, Gerüstproteine.

Ein weiterer Gegenstand der vorliegenden Erfindung bezieht sich auch auf ein Diagnostikum enthaltend ein erfindungsgemäßes Linker-Nucleosid, eine erfindungsgemäße Nucleinsäure oder ein erfindungsgemäßes Konjugat, wie oben bereits näher beschrieben.

Ein anderer Gegenstand der Erfindung ist daher die Verwendung eines erfindungsgemäßen Linker-Nucleosids, einer erfindungsgemäßen Nucleinsäure, eines erfindungsgemäßen Konjugates und/oder eines erfindungsgemäßen Trägers zur Herstellung eines Arzneimittels, wie z. B. eines Therapeutikums, eines Diagnostikums und/oder eines elektronischen Bauteils.

Die Erfindung bezieht sich auch auf die Verwendung der erfindungsgemäßen Linker-Nucleoside, der erfindungsgemäßen Nucleinsäure bzw. des erfindungsgemäßen Konjugates und/oder des erfindungsgemäßen Trägers in einem Paarungs- und/oder Testsystem, wie z. B. in WO94/28173, WO96/13522, WO96/13613, R. L. Letsinger, et al., Nature, 1996, 382, 607-9; P. G. Schultz et al., Nature, 1996, 382, 609-11 oder A. Lombardi, J. W. Bryson, W. F. DeGrado, Biopolymers (Pept. Sci.) 1997, 40, 495-504 näher beschrieben und oben allgemein erläutert.

Die folgenden Figuren und Beispiele sollen die Erfindung näher beschreiben, ohne sie zu beschränken.

BESCHREIBUNG DER FIGUREN

Fig. 1 zeigt einen Ausschnitt aus der Struktur von RNA in ihrer natürlich vorkommenden Form (links) und in Form einer p-NA (rechts).

Fig. 2 zeigt schematisch die Synthese eines p-Ribo(A,U)-Oligonucleotids nach Eschenmoser et al. (1993).

Fig. 3 zeigt schematisch eine Anordnung von immobilisierten Erkennungsstrukturen (Arrays) auf einem festen Träger.

## BEISPIELE

### Synthese von p-RNA-Linkersystemen

Im folgenden werden drei Wege beschrieben, die die Bereitstellung von Linkern, die einen Amino-Terminus aufweisen, der dann zum Anlinken funktioneller Einheiten verwendet werden kann, ermöglichen:

### <u>Beispiel 1</u>

### Uracil-basierender Linker

auf der Basis der Modifizierung der 5-Position des Uracils.

Die Herstellung von Hydroxyethyluracil **28** gelingt im großen Maßstab nach bekannter Methode (J.D. Fissekis, A. Myles, G.B. Brown, J. Org. Chem. 1964, 29, 2670. g-Butyrolacton **25** wurde mit Ameisensäuremethylester formyliert, das Natriumsalz **26** zum Harnstoffderivat **27** umgesetzt und dieses zum Hydroxyethyluracil **28** cyclisiert (Schema 1).

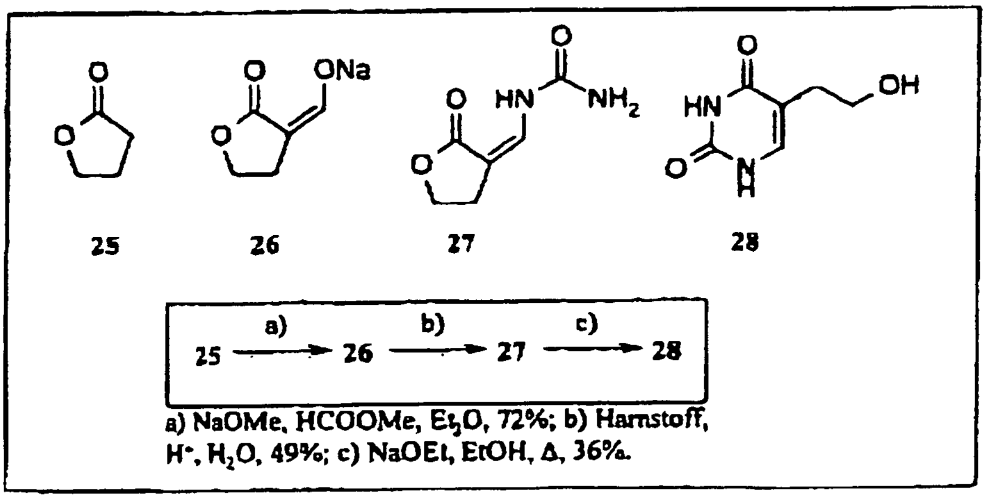


Schema 1: Synthese von Hydroxyethyluracil **28**.

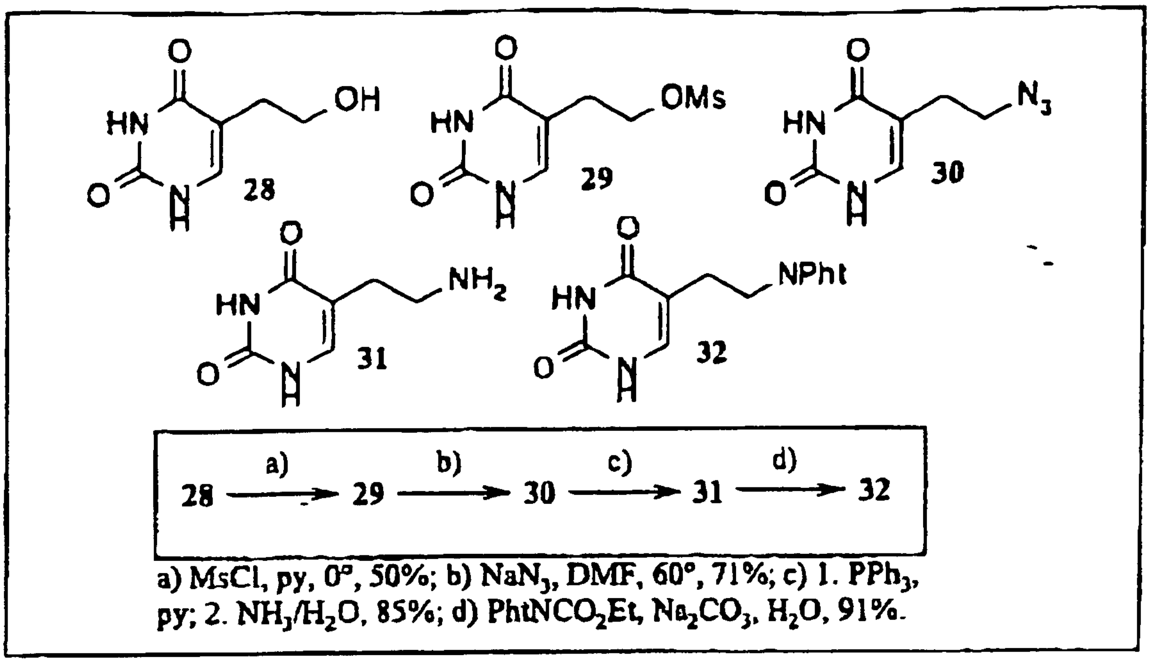


Schema 2: Synthese von N-Phtaloylaminoethyluracil **32**.

Hydroxyethyluracil **28** wurde mit Methansulfonsäurechlorid in Pyridin mesyliert zu **29** (J.D. Fissekis, F. Sweet, J. Org. Chem. 1973,38,264).
Die folgenden Stufen wurden neu erfunden: Mit Natriumazid in DMF wurde **29** zum Azid **30** umgesetzt und dieses mit Triphenylphosphin in Pyridin zum Aminoethyluracil **31** reduziert. Die Aminofunktion in **31** wurde schließlich mit N-Ethoxycarbonylphtalimid geschützt (Schema 2). Nukleosidierung von Ribosetetrabenzoat **33** mit N-Phtaloylaminoethyluracil **32** lieferte das Ribosetribenzoat **34** in guten Ausbeuten. Das anomere Zentrum des Pyranoseringes ist, wie aus der Kopplungskonstanten zwischen H-C(1') und H-C(2') von J = 9.5 Hz klar ersichtlich, β-konfiguriert. Anschließende Abspaltung der Benzoatschutzgruppen mit NaOMe in MeOH lieferte das Linkertriol 35.35 wurde bei -78°C in Pyridin/Dichlormethan 1:10 in Gegenwart von DMAP mit Benzoylchlorid umgesetzt. Dabei erhielt man neben dem gewünschten 2'-Benzoat **36** (64%) auch 2',4'-dibenzoyliertes Produkt (22%), welches gesammelt und analog der Methanolyse von **34** nach 35 wieder in das Triol **35** umgewandelt wurde. Das 2'-Benzoat **36** wurde mit Dimethoxytritylchlorid in Gegenwart von Hünig-Base in Dichlormethan in 4'-Position in Ausbeuten größer 90% trityliert. Die Umlagerung von 4'-DMT-2'-benzoat **37** zum 4'-DMT-3'-benzoat **38** erfolgte in Gegenwart von DMAP. p-Nitrophenol und Hünig-Base in n-Propanol/Pyridin 5:2. Nach Chromatographie wird **38** erhalten. 4'-DMT-3'-benzoat **38** wurde abschließend mit $ClP(OAll)N(iPr)_2$ in Gegenwart von Hünig-Base zum Phosphoramidit **39** umgesetzt (Schema 3). Dieser läßt sich ohne Änderung der Syntheseprotokolle für die automatisierte Oligonucleotidsynthese einsetzen.

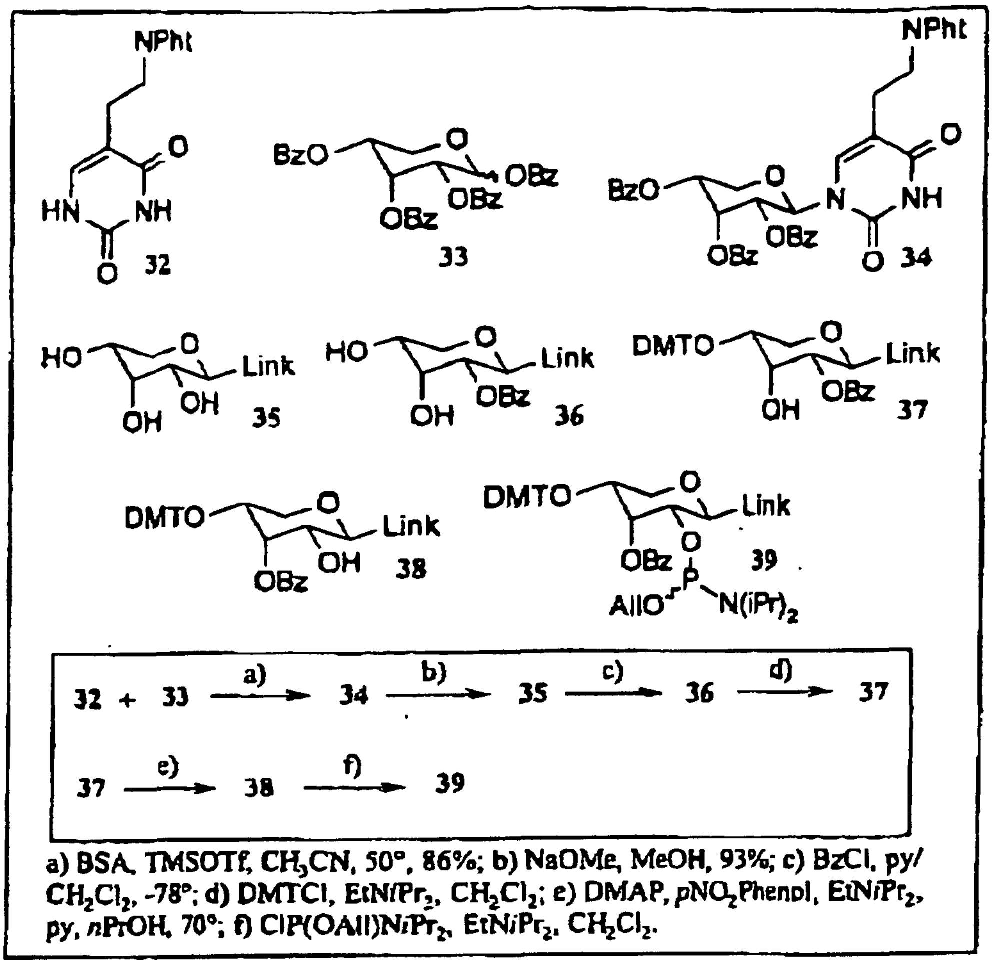


**Schema 3: Synthese des Linkerphosphoramidites 39.**

Ausführung:

Synthese eines Uracil-Linker-Hausteins

**5-(2-Azidoethyl)uracil (30)**

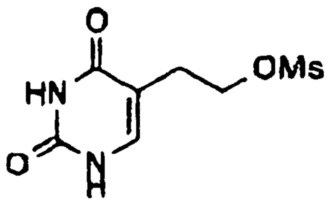
29

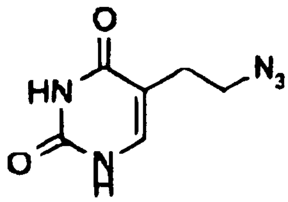
30

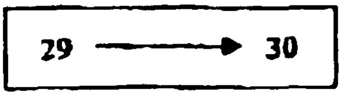


## 1. Durchführung

In einem mit Innenthermometer und Rückflußkühler ausgestatteten 500-ml-Dreihalskolben wurden 26.0 g (0.11 mol) **29** in 250 ml DMF gelöst und mit 10.8 g (0.166 mol) Natriumazid versetzt. Die Suspension wurde im Anschluß vier Stunden bei 60°C gerührt (DC-Kontrolle. $CHCl_3$/MeOH 9: 1). Das DMF wurde abdestilliert und der Rückstand mit 150 ml Wasser verrührt. Der Feststoff wurde abfiltriert, mit ca. 50 ml Wasser gewaschen und über Nacht im Vakuum im Exsiccator über Phosphorpentoxid getrocknet. Man erhielt 14.2 g (71%) 30 in Form eines farblosen Feststoffes vom Schmp. 230-235°C (u. Zers.).

## 2. Analytische Daten

5-(2-Azidoethyl)uracil (30):

| | |
|---|---|
| **Schmp.:** | 230-235°C u. Zersetz.. |
| **DC:** | $CHCl_3$/MeOH 9:1, $R_f$ 0.48. |
| **UV** (MeOH): | $\lambda_{max}$ 263.0 (7910). |
| **IR** (KBr): | 3209s, 3038s, 2139s, 1741s, 1671 s, 1452m, 1245m. 1210m. |
| **$^1$H-NMR** (300 MHz, $d_6$-DMSO): | 2.46 (t, 2H, J($CH_2CH_2N$, $CH_2CH_2N$) = 7.0, $CH_2CH_2N$); 3.40 (t, 2H, J($CH_2CH_2N$, $CH_2CH_2N$) = 7.0. $CH_2CH_2N$); 7.36 (s, H-C(6)); 11.00 (br. s, 2H. H-N(1), H-N(3)). |
| **MS** (ESI$^+$): | 180.0 [M+H].. |

## 5-(2-Aminoethyl)uracil (31)

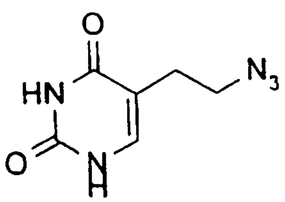
30

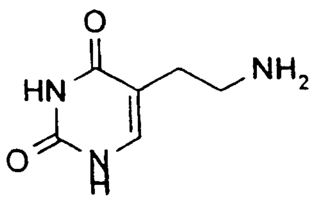
31

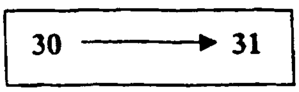

### 1. Durchführung

In einem mit Innenthermometer und Rückflußkühler ausgestatteten 250-ml-Dreihalskolben wurden 14.2 g (78.0 mmol) **30** in 175 ml Pyridin suspendiert und mit 61.4 g (234 mmol) Triphenylphosphin versetzt[2]. Es wurde fünf Stunden auf 60°C erwärmt und über Nacht bei Raumtemp. gerührt (DC-Kontrolle, $CHCl_3$/MeOH 5:1). Zur Suspension wurden 40 ml einer 25%igen Ammoniaklösung gegeben, die daraufhin aufklarte. Die Lösungsmittel wurden i.v.i.RV entfernt. Der Rückstand wurde in 200 ml $CH_2Cl_2$/MeOH 1:1 30 min bei Raumtemp. gerührt, der Niederschlag abfiltriert und mit $CH_2Cl_2$ gewaschen. Nach Trocknen im Vakuum im Exsiccator über Phosphorpentoxid erhielt man 10.0 g (85%) **31** vom Schmp. 214-220°C.

### 2. Analytische Daten

5-(2-Aminoethyl)uracil (**31**):

| | |
|---|---|
| **Schmp.:** | 214-220°C u. Gasentwicklung, vorher sintern. |
| **DC:** | $CHCl_3$/MeOH/HOAc/$H_2O$ 85:20:10:2, $R_f$ 0.07. |
| **UV** (MeOH): | $\lambda_{max}$ 263.0 (6400). |
| **IR** (KBr): | 3430m, 3109s, 1628s, 1474m, 1394s, 1270s, 1176w, 1103m, 1021m, 966m, 908m, 838m. |
| **$^1$H-NMR** (300 MHz, $d_6$-DMSO): | 2.21 (t, 2H. J($CH_2CH_2N$, $CH_2CH_2N$) = 6.8. $CH_2CH_2N$); 2.59 (t, 2H, J($CH_2CH_2N$, $CH_2CH_2N$) = 6.8, $CH_2CH2_N$); 5.90 (v. br. s. 4H, H-N(1), H-N(3), $NH_2$); 7.19 (s, H-C (6)). |
| **MS** (ESI$^-$): | 153.9 [M-H]. |

**5-(2-Phtalimidoethyl)uracil (32)**

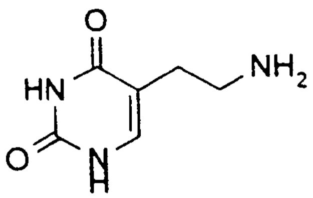

31

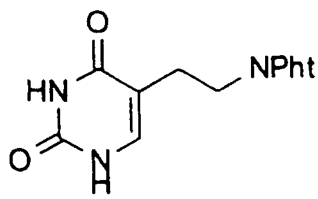

32

31 → 32

### 1. Durchführung

In einem 250-ml-Rundkolben wurden 9.6 g (61.8 mmol) **31** in 100 ml Wasser suspendiert und mit 6.64 g (62.6 mmol) $Na_2CO_3$ versetzt. Nach 15 min Rühren bei Raumtemp. gab man portionsweise 14.3 g (65 mmol) N-Ethoxycarbonylphtalimid zu und rührte drei Stunden bei Raumtemp. (DC-Kontrolle, $CHCl_3$/MeOH 5:1). Die nunmehr zähe, weiße Suspension wurde vorsichtig[1] mit konz. Salzsäure auf pH 4 eingestellt und der weiße Niederschlag abfiltriert. Nach Waschen mit Wasser wurde der Feststoff im Vakuum im Exsiccator über Phosphorpentoxid getrocknet. Dies lieferte 16.0 g (91%) 32 vom Schmp. 324-327°C.

## 2. Analytische Daten

5-(2-Phtalimidoethyl)uracil (32):

| | |
|---|---|
| **Schmp.:** | 324-327°C u. Zersetz.. |
| **DC:** | $CHCl_3$/MeOH 5:1, $R_f$ 0.51. |
| **UV** (MeOH): | $\lambda_{max}$ 263.0 (5825); $\lambda$ 298.0 (sh., 1380). |
| **IR** (KBr): | 3446m, 3216m, 1772m, 1721s, 1707s, 1670s, 1390m. |
| **$^1$H-NMR** (300 MHz, $d_6$-DMSO): | 2.49 (t, 2H, J($CH_2CH_2N$, $CH_2CH_2N$) = 6.0, $CH_2CH_2N$); 3.71 (t, 2H, J($CH_2CH_2N$, $CH_2CH_2N$) = 6.0, $CH_2CH_2N$); 7.24 (s, H-C(6)); 7.84 ($m_c$, 4H, NPht); 10.76 (br. s, H-N(1), H-N(3)). |
| **MS** (ESI$^-$): | 284.0 [M-H]. |

**1-(2, 3, 4-Tri-O-benzoyl-β-D-ribopyranosyl)-5-(2-phtalimido-ethyl)uracil (34)**

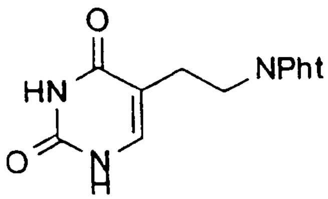

32

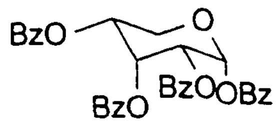

33

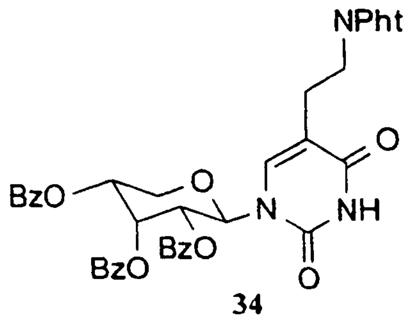

34

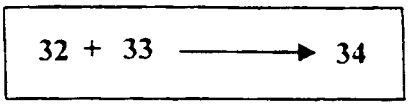


## 1. Durchführung

In einem 250-ml-Dreihalskolben, ausgestattet mit Argonüberleitung, Innenthermometer und Septum, wurden 7.00 g (24 mmol) **32** und 13.6 g (24 mmol) **33** in 120 ml Acetonitril suspendiert. Dann wurden mittels Spritze zunächst 12.2 ml (50 mmol) BSA zugegeben und nach 30 min Rühren nochmals 7 ml (28 mmol) BSA zugegeben. Nach kurzzeitigem Erwärmen auf 40°C klarte sich die Reaktionsmischung auf. Bei Raumtemp. wurden 13 ml (72 mmol) TMSOTf mittels Spritze zugegeben. Nach einer Stunde konnte noch keine Produktbildung beobachtet werden (DC-Kontrolle, AcOEt/n-Heptan 1:1). Daher wurden weitere 13 ml (72 mmol) TMSOTf zugegeben. Im Anschluß wurde der Reaktionsansatz auf 50°C erwärmt. Nach 2.5 h Rühren bei 50°C (DC-Kontrolle) wurde auf RT. gekühlt, auf eine eiskalte Mischung von 250 ml AcOEt und 190 ml ges. $NaHCO_3$-Lösung und 10 min intensiv ausgerührt. Man wusch nochmals mit 100 ml $NaHCO_3$-Lösung und extrahierte die wäßrigen Phasen nochmals mit 100 ml AcOEt. Die ver. org. Phasen wurden mit $MgSO_4$ getrocknet und die Lösungsmittel i.V.i.RV entfemt. Nach Trocknen im ÖPV erhielt man 20.9 g Rohprodukt. Chromatographie an Kieselgel (h = 25 cm, ⌀= 5 cm, AcOEt/n-Heptan 1:1) lieferte ein DCeinheitliches, schaumiges Produkt, das mit $Et_2O$ digeriert wurde. Filtration und Trocknen im ÖPV ergab 15 g (86%) **34**.

## 2. Analytische Daten

**1-(2, 3, 4-Tri-O-benzoyl-β-D-ribopyranosyl)-5-(2-phtalimidoethyl)uracil (34):**

| | |
|---|---|
| **Schmp.:** | 124°C (sintern). |
| **DC: UV** (MeOH): | AcOEt/n-Heptan 1:1, $R_f$ 0.09. $\lambda_{max}$ 263.0 (11085); $\lambda$ 299.0 (sh., 1530). |
| **IR** (KBr): | 3238w, 3067w, 1772m, 1710s, 1452m, 1395m, 1266s, 1110s, 1070m, 1026m. |
| **$^1$H-NMR** (300 MHz, $CDCl_3$): | 2.79 ($m_c$, 2H, $CH_2CH_2N$); 3.96 ($m_c$, 2H, $CH_2CH_2N$); 4.06 (dd, J($H_{eq}$-C(5'), $H_{ax}$-C(5')) = 11.0, J($H_{eq}$-C(5'), H-C(4')) = 6.0, $H_{eq}$-C(5')); 4.12 (t, J($H_{ax}$-C(5'), $H_{eq}$-C(5')) = J($H_{ax}$-C (5'), H-C(4')) = 11.0, $H_{ax}$-C(5')); 5.39 (dd, J(H-C(2'), H-C(1')) = 9.5, J(H-C(2'), H-C(3')) = 2.9, H-C(2')); 5.46 (ddd, J(H-C(4'), $H_{ax}$-C(5')) = 11.0, J(H-C(4'), $H_{eq}$-C(5')) = 6.0. J (H-C(4'), H-C(3')) = 2.9, H-C(4')); 6.26 ($\psi$t, J $\approx$ 2.6, H-C(3')); 6.36 (d, J(H-C(1'), H-C (2')) = 9.5, H-C(1')); 7.24-7.40, 7.44-7.56, 7.61-7.66, 7.72-7.80, 7.84-7.90, 8.06-8.13 (6m, 16H, 3 Ph, H-C(6)); 7.70, 7.82 (2 $m_c$. 4H, NPht); 8.37 (s, H-N(3)). |
| **$^{13}$C-NMR** (75 MHz. $CDCl_3$): | 21.19 ($CH_2CH_2N$); 36.33 ($CH_2CH_2N$); 64.07 (C(5')); 66.81, 68.22 (C(4'), C(2')); 69.29 (C(3')); 78.59 (C(1')); 112.42 (C(5)); 123.31. 132.05, 133.89 (6C, Pht); 128.33, 128.47, 128.47, 128.83, 128.86, 129.31, 129.83, 129.83, 129.94, 133.55, 133.62, 133.69 (18C, 3 Ph); 135.87 (C(6)); 150.39 (C(2)); 162.78 (C(4)); 164.64, 165.01, 165.41 (3C, $O_2CPh$); 168.43 (2C, CO-Pht). |
| **MS** (ESI$^+$): | 730.2 [M+H]. |
| **Anal.:** | ber. für $C_{40}H_{31}N_3O_{11}$ (729.70): C 65.84, H 4.28, N 5.76; gef.: C 65.63, H 4.46, N 5.53. |

**5-(2-Phtalimidoethyl)-1-(β-D-ribopyranosyl)uracil (35)**

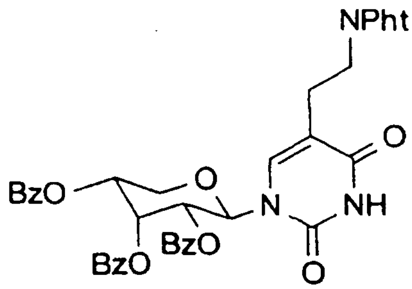

**34**

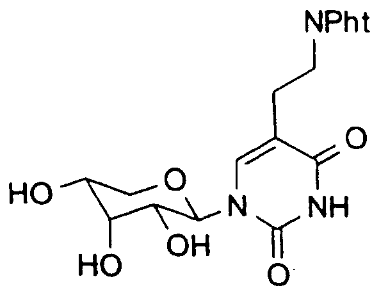

**35**

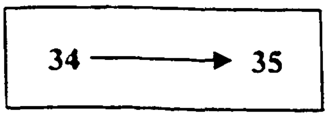


### 1. Durchführung

In einem 1-1-Rundkolben wurden 15 g (20 mmol) **34** in 500 ml MeOH gelöst, mit 324 mg (6 mmol) NaOMe versetzt und über Nacht unter Wasserausschluß bei Raum-temp. gerührt (DC-Kontrolle, AcOEt/n-Hepcan 1:1). Es wurde solange Amberlite IR-120 zur entstandenen Suspension gegeben bis der pH-Wert <7 war. Es wurde der Feststoff in der Hitze gelöst, heiß vom Ionentauscher abfiltriert und mit MeOH gewaschen. Nach Entfernen der Lösungsmittel wurde der Rückstand zweimal mit je 150 ml Wasser coevaporiert. Dies lieferte 9 g Rohprodukt, das 10 min in 90 ml MeOH unter Rückfluß erhitzt wurde. Nach Abkühlen auf Raumtemp. versetzte man mit 60 ml $Et_2O$ und bewahrte über Nacht bei 4°C auf. Filtration, Waschen mit $Et_2O$ und Trocknen im ÖPV lieferte 7.8 g (93%) 35.

### 2. Analytische Daten

5-(2-Phtalimidoethyl)-1-(β-D-ribopyranosyl)uracil (**35**):

| | |
|---|---|
| **Schmp.:** | 137°C (sintern). |
| **DC:** | $CHCl_3$/MeOH 5:1, $R_f$ 0.21. |
| **UV** (MeOH): | $\lambda_{max}$ 263.0 (8575); $\lambda$ 299.0 (sh., 1545). |
| **IR** (KBr): | 3458s. 1772w, 1706s. 1400m, 1364m, 1304m, 1045m. |
| **$^1$H-NMR** (300 MHz. $d_6$-DMSO + 2 Tr. $D_2O$). | 2.55 ($m_c$. 2H. $CH_2CH_2N$); 3.28-3.61 (m. 4H, H-C(2'), H-C(4'), $H_{eq}$-C(5'). $H_{ax}$-C(5)): 3.73 ($m_c$, 2H. $CH_2CH_2N$): 3.93 (m, H-C(3')); 5.50 (d, J(H-C (1'), H-C(2')) = 9.3, H-C(1')); 7.41 (s, H-C(6)); 7.84 (s, 4H, NPht). |
| **$^{13}$C-NMR** (75 MHz, $d_6$-DMSO): | 25.63 ($CH_2CH_2N$); 36.62 ($CH_2CH_2N$); 64.95 (C(5')); 66.29 (C(4')); 67.37 (C(2')); 71.12 (C(3')); 79.34 (C(1')); 110.39 (C(5)); 122.85. 131.54, 134.08 (6C, Pht); 137.92 (C(6)); 150.84 (C(2)); 163.18 (C(4)); 167.74 (2C, CO-Pht). |
| **MS** ($ESI^-$): | 416.1 [M-H]. |

**1-(2'-O-Benzoyl-β-D-ribopyranosyl)-5-(2-phtalimidoethyl)-uracil**

In einem ausgeheizten und mit Argon begasten 1-1-Vierhalskolben wurden 10.6 g (0.025 mmol) 5-(2-Phtalimidoethyl)-1-(β-D-ribopyranosyl)uracil in 20 ml Pyridin gelöst und mit 200 ml Dichlormethan versetzt. Es wurde auf -70°C abgekühlt, unter Kühlung 3.82 ml (0.033 mmol) Benzoylchlorid in 5 ml Pyridin und 20 ml Dichlormethan langsam zugetropft und 35 min bei -70°C gerührt. Das Reaktionsgemisch wurde auf 600 ml gekühlte Ammoniumchlorid-Lösung gegossen und die wäßrige Phase mit Ethylacetat extrahiert. Die vereinigten organischen Phasen wurden mit Wasser gewaschen, getrocknet und im Vakuum zur Trockene eingeengt. Chromatographie über Kieselgel (Ethylacetat/Heptan 1:1) lieferte 7.9 g (60%) 1-(2'-O-Benzoyi-β-D-ribopyranosyl)-5-(2-phtalimidoethyl)-uracil. DC: $R_f$ 0.24 (Ethylacetat/Heptan 4:1). $^1$H-NMR (300 Mhz, $d_6$-DMSO): 2.67 ($m_c$ 2H, $CH_2CH_2N$); 3.66-3.98 (m. 5H, H-C(4'), $H_{eq}$-C(5'), $H_{ax}$-C(5'), $CH_2CH_2N$); 4.51 (t, 1H, H-C(3')); 4.98 (dd, 1 H, H-C(2')); 6.12 (d, 1 H, H-C(1')); 7.19 (s, 1H, H-C(6)); 7.29-7.92 (m, 9H, OBz, NPht).

**1-(2-O-Benzoyl-4-O-(4,4'-dimethoxytrityl)-β-D-ribopyranosyl)-5-(2-phtalimidoethyl)uracil**

5.6 g (10.73 mmol) 1-(2-O-Benzoyl-β-D-ribopyranosyl)-5-(2-phtalimidoethyl)uracil wurden in 60 ml Dichlormethan gelöst, mit 4.72 g (13.95 mmol) 4,4'-Dimethoxytritylchlorid und 2.4 ml (13.95 mmol) N-Ethyl-diisopropylamin versetzt und 20 min bei RT gerührt. Das Reaktionsgemisch wurde mit 100 ml Dichlormethan verdünnt, mit Natriumhydrogencarbonat-Lösung und 20% Zitronensäure-Lösung gewaschen, getrocknet und im Vakuum zur Trockene eingeengt. Chromatographie über Kieselgel (Ethylacetat/Heptan 1:1 + 2% Triethylamin) lieferte 7.7 g (87%) 1-(2-O-Benzoyl-4-O-(4,4'-dimethoxytrityl)-β-D-ribopyranosyl)-5-(2-phtalimidoethyl)uracil.
DC: $R_f$ 0.53 (Ethylacetat/Heptan 1:1 + 2% Triethylamin).
$^1$H-NMR (300 MHz. $CDCl_3$,): 2.64 ($m_c$, 2H, $CH_2CH_2N$); 3.12 ($m_c$, 1H, H-C(4')); 3.59-3.63 und 3.72-3.92 (m, 5H, H-C(3'), $H_{eq}$-C(5'), $H_{ax}$-C(5'), $CH_2CH_2N$); 3.81 und 3.82 (s, 6H, $CH_3O$); 4.70 (dd, 1H, H-C(2')); 6.09 (d, 1H, H-C(1')); 7.05 (s, 1H, H-C(6)); 6.84-7.90 (m, 22H, ODmt, OBz, NPht).

**1-(3-O-Benzoyl-4-O-(4,4'-dimethoxytrityl)-β-D-ribopyranosyl)-5-(2-phtalimidoethyl)uracil**

3 g (3.63 mmol) 1-(2-O-Benzoyl-4-O-(4,4'-dimethoxytrityl)-β-D-ribopyranosyl)-5-(2-phtalimidoethyl)uracil. 1 g (7.26 mmol) 4-Nitrophenol, 0.44 g (3.63 mmol) 4-(Dimethylamino)-pyridin und 3.75 ml (21.78 mmol) N-Ethyl-diisopropylamin wurden in 5.6 ml iso-Propanol und 25 ml Pyridin gelöst, auf 65°C erhitzt und 3 Tage bei 65°C gerührt. Die Lösung wurde im Vakuum zur Trockene eingeengt und der Rückstand in 150 ml Dichlormethan gelöst. Nach Waschen mit 20% Zitronensäure-Lösung und Natriumhydrogencarbonat-Lösung wurde über Magnesiumsulfat getrocknet. Chromatographie über Kieselgel (Ethylacetat/Dichlormethan/iso-Hexan 2:1:1) lieferte 2.27 g (76%) 1-(3-O-Benzoyl-4-O-(4,4'-dimethoxytrityl)-β-D-ribopyranosyi)-5-(2-phtalimidoethyl)uracil.
DC: $R_f$ 0.27 (Ethylacetat/iso-Hexan 2:1 + 1% Triethylamin).
$^1$H-NMR (300 MHz, $CDCl_3$): 2.39 ($m_c$, 2H, $CH_2CH_2N$); 2.53 ($m_c$, 1H, $H_{eq}$-C(5')); 3.30 (dd. 1H, H-C(2')); 3.55 ($m_c$, 1H, $H_{ax}$-C(5')); 3.69 ($m_c$, 2H. $CH_2CH_2N$); 3.78 und 3.79 (s, 6H. $CH_3O$); 3.79-3.87 (m, 1H, H-C(4')); 5.74 (d, 1H, H-C(1')); 5.77 ($m_c$, 1H, H-C(3')); 6.92 (s, 1H, H-C(6)); 6.74-8.20 (m, 22H, ODmt, OBz, NPht).

**1-{2'-O-[(Allyloxy)(diisopropylamino)phosphino]-3'O-benzoyl-4'-O-[(4,4'-dimethoxytriphenyl)-methyl]-β-D-ribo-pyranosyl}-5-(2-phtalimidoethyl)- uracil**

88 mg (0.11 mmol) 1-(3-O-Benzoyl-4-O-(4,4'-dimethoxytrityl)-β-D-ribopyranosyl)-5-(2-phtalimidoethyl)uracil wurden in 5 ml Dichlormethan gelöst, mit 75 µl (0.44 mmol) N-Ethyldiisopropylamin und 70 µl (0.3 mmol) Allyloxy-chlor-(diisopropylamino)phosphin versetzt und 3 h bei Raumtemperatur gerührt. Nach Zugabe von weiteren 35 µl (0.15 mmol) Allyloxychlor-(diisopropylamino)phosphin zur Vervollständigung der Reaktion wurde noch 1 h bei Raumtemperatur gerührt und das Reaktionsgemisch im Vakuum eingeengt. Chromatographie an Kieselgel (Ethylacetat/Heptan: Gradient 1:2 auf 1:1 1 auf 2:1, jeweils mit 2% Triethylamin) lieferte 85 mg (76%) 1-{2'-O-[(Allyloxy)(diisopropylamino)phosphino)-3'O-benzoyl-4'-O-[(4,4'-dimethoxytriphenyl)-methyl)-β-D-ribo-pyranosyl}-5-(2-phtalimidoethyl)- uracil.
DC: $R_f$ 0.36 (Ethylacetat/Heptan 2:1).
$^1$H-NMR ($CDCl_3$, 300 MHz): Ausgewählte charakteristische Lagen: 2.28, 2.52 (2 dd, J = 5.0, 11.0 Hz, 2 H, 2 H-5'), 3.79, 3.78 (app. 2 s, 12 H, OMe), 6.14 (1 bs, 1 H, H-3'). $^{31}$P-NMR ($CDCl_3$): 149.8, 150.6

Beispiel 2

**Indol-basierender Linker**

N-Phthaloyltryptamin wird wie beschrieben aus Phthalsäureanhydrid und Tryptamin erhalten (Kuehne et al J. Org. Chem. **43**, 13, **1978**, 2733-2735). Dieses wird mit Boran-THF zum Indolin reduziert (analog A. Giannis, et al., Angew. Chem. 1989, 101, 220).

Das 3-substituierte Indolin wird zuerst mit Ribose zum Nucleosidtriol und dann mit Essigsäureanhydrid zum Triacetat umgesetzt. Man oxidiert mit 2,3-Dichlor-5,6-dicyanoparachinon und spaltet die Acetate mit Natriummethylat, benzoyliert selektiv in 2'-Position, DM-trityliert selektiv in 4'-Position, und führt die Wanderungsreaktion zum 3'-Benzoat durch. Die Bildung des Phosphoramidits erfolgt wie üblich. Dieser läßt sich ohne Änderung der Syntheseprotokolle für die automatisierte Oligonucleotidsynthese einsetzen.

Durchführung

**3-(N-Phthaloyl-2-aminoethyl)-indolin**

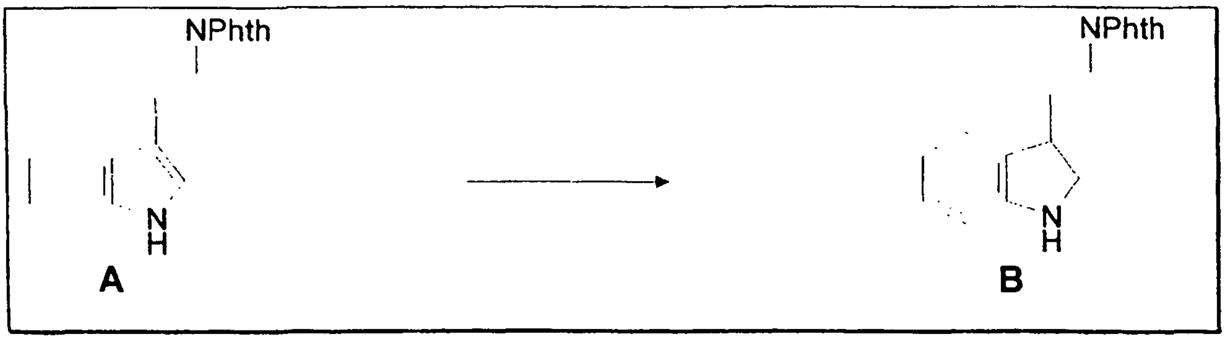

Unter Stickstoffatmosphäre wurden 51.4 g (177 mmol) Phthaloyltryptamin A in 354 ml 1M Boran-THF-Lösung (2 eq.) gelöst und auf 0 °C abgekühlt. Bei 0 °C wurde langsam 354 ml Trifluoressigsäure zugetropft (Vorsicht: Gasentwicklung) und 30 min. nachgerührt (DC-Kontrolle: EtOAc). Dann wurden 17.3 ml Wasser zugegeben, 10 min gerührt und im Vak. eingeengt. Der Rückstand wurde in 10%iger NaOH-Lösung / Dichlormethan gelöst, die organische Phase wurde abgetrennt, über $NaSO_4$ getrocknet, filtriert und im Vak. eingeengt. Der Rückstand (50.9 g) wurde aus heißem Ethanol (3 1) umkristallisiert. Man erhielt 41.4 g **B**, Smp. 161-162°C. Die Mutterlauge wurde im Vakuum eingeengt und der Rückstand erneut aus Ethanol umkristallisiert. Man erhielt weitere 3.2 g **B**, Smp. 158-159 °C.
Gesamtausbeute: 44.6 g (153 mmol) **B**, d. s. 86%.
$^1$H-NMR ($CDCl_3$, 300 MHz): 1.85-2.00, 2.14-2.28 (2 m, 2 x 1 H, C$\underline{H_2}$CH$_2$NPhth), 2.70 (bs, 1 H, NH), 3.24-3.38, 3.66-3.86 (2 m, 5 H, CH$_2$C$\underline{H_2}$NPhth, H-2a, H-2b, H-3), 6.62 (d, J = 8.0 Hz, 1 H, H-7), 6.66-6.72 (m, 1 H, H-5), 6.99 (app t, J = 7.5 Hz, 1 H, H-6), 7.14 (d, J = 8.0 Hz, 1 H, H-4), 7.64-7.74, 7.78-7.86 (2 m, 2 x 2 H, Phth).
$^{13}$C-NMR ($CDCl_3$, 75 MHz): 32.70, 36.10 (2 t, C-2, $\underline{C}$H$_2$CH$_2$NPhth), 39.62 (d, C-3), 53.04 (t, $\underline{C}$H$_2$NPhth), 109.65 (d, C-7), 118.74 (d, C-5), 123.25 (d, Phth), 123.92, 127.72 (2 d, C-4, C-6), 131.81 (s, C-3a), 132.14 (s, Phth), 133.99 (d, Phth), 151.26 (s, C-7a), 168.38 (s, C=O).
Ber: C: 73.96, H: 5.52, N: 9.58; gef.: C: 73.89, H: 5.57, N: 9.55.
MS ($ES^+$): 293 ($MH^+$, 100%)

**3-(N-Phthaloyl-2-aminoethyl)-1-(2',3',4'-tri-O-acetyl-β-D-ribo-pyranosyl)-indol**

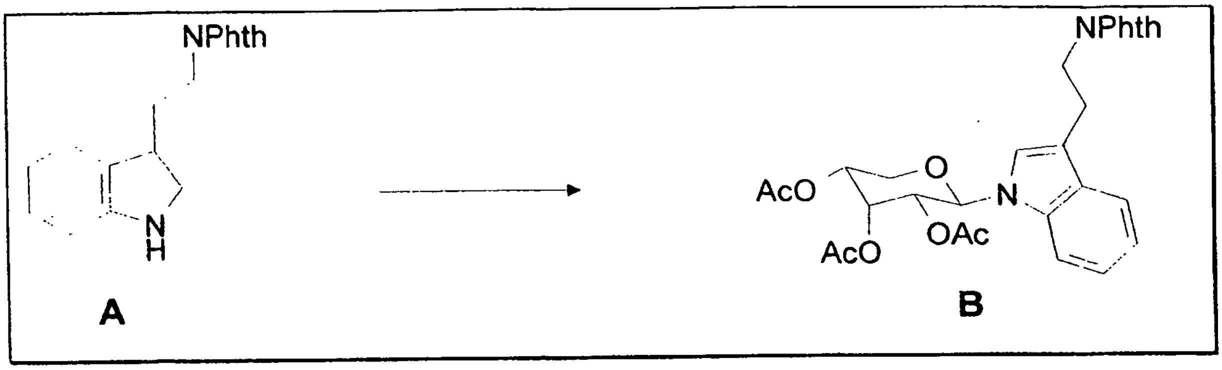

Unter Stickstoffatmosphäre wurden 45.2 g (155 mmol) A und 23.2 g (155 mmol; 1.0 eq.) D-Ribose in 750 ml trockenem Ethanol suspendiert und 4 h zum Rückfluß erhitzt (DC-Kontrolle: $CH_2Cl_2$/MeOH 10:1). Nach Abkühlen auf RT wurde im Vak. eingeengt. Der Rückstand wurde in 300 ml Pyridin gelöst und unter Eiskühlung mit 155 ml Essigsäureanhydrid versetzt. Nach 15 min. wurde das Eisbad entfernt und 18 h bei RT gerührt (DC-Kontrolle: EtOAc/iso-Hexan 1:1). Diese Lösung wurde im Vakuum eingeengt und 3 mal mit je 300 ml Toluol coevaporiert. Das erhaltene Öl wird in 900 ml Dichlormethan gelöst und unter Eiskühlung mit 38.8 g (171 mmol; 1.1 eq.) 2,3-Dichlor-5,6-dicyanoparachinon versetzt. Nach 15 min. wurde das Eisbad entfernt und 1.5 h bei RT nachgerührt (DC-Kontrolle: EtOAc/iso-Hexan 1:1). Der ausgefallene Niederschlag wurde abgesaugt und mit Dichlormethan gewaschen und verworfen. Das Filtrat wurde mit 600 ml ges. $NaHCO_3$-Lösu gewaschen. Der dabei ausgefallene Niederschlag wurde erneut abgesaugt und mit Dichlormethan gewaschen und verworfen. Die vereinigten organischen Extrakte wurden über $NaSO_4$ getrocknet und im Vak. eingeengt. Der Rückstand (90.9 g) wurde durch Flashchromatographie an Kieselgel 60 gereinigt (10 x 25 cm; EtOAc/iso-Hexan 2:3).
Man erhielt: 21.5 g reines **B** und 46.83 g Mischfraktionen, die nach erneuter Chromatographie weitere 20.4 g reines **B** lieferten.
Gesamtausbeute: 41.9 g (76 mmol) **B**, d. s. 49%.
$^1$H-NMR ($CDCl_3$, 300 MHz): 1.64, 1.98, 2.19 (3 s, 3 x 3 H, Ac), 3.06 (t, J = 8.0 Hz, 2 H, C$\underline{H_2}$$CH_2$NPhth), 3.81-4.00 (m, 4 H, H-5'ax, H-5'eq, C$\underline{H_2}$NPhth), 5.13 (ddd, J = 2.5, 6.0, 10.5 Hz, 1 H, H-4'), 5.36 (dd, J = 3.5, 9.5 Hz, 1 H, H-2'), 5.71 (d, J = 9.5 Hz, 1 H, H-1'), 5.74 (app t, J = 3.0 Hz, 1 H, H-3'), 7.02 (s, 1 H, H-2), 7.04-7.10, 7.13-7.19 (2 m, 2 x 1 H, H-5, H-6), 7.33 (d, J = 8.0 Hz, 1 H, H-7), 7.58-7.66, 7.72-7.80 (2 m, 5 H, Phth, H-4).
$^{13}$C-NMR ($CDCl_3$, 75 MHz): 20.23, 20.65, 20.87 (3 q, Ac), 24.41, 38.28 (2 t, $CH_2CH_2$), 63.53 (t, C-5'), 66.24, 68.00, 68.64 (3 d, C-2', C-3', C-4'), 80.33 (d, C-1'), 109.79 (d, C-7), 113.95 (s, C-3), 119.33, 120.39, 122.04, 122.47 (4 d, C-4, C-5, C-6, C-7), 123.18 (d, Phth), 128.70, 132.17 (2 s, C-3a, Phth), 133.87 (d, Phth), 136.78 (s, C-7a), 168.24, 168.77, 169.44, 169.87 (4 s, C=O).
Ber: C: 63.50, H: 5.15, N: 5.11; gef.: C: 63.48, H: 5.16, N: 5.05.
MS ($ES^+$): 566 ($M+NH_4^+$, 82%), 549 ($MH^+$, 74%), 114 (100%).

**3-(N-Phthaloyl-2-aminoethyl)-1-β-D-ribo-pyranosyl-indol**

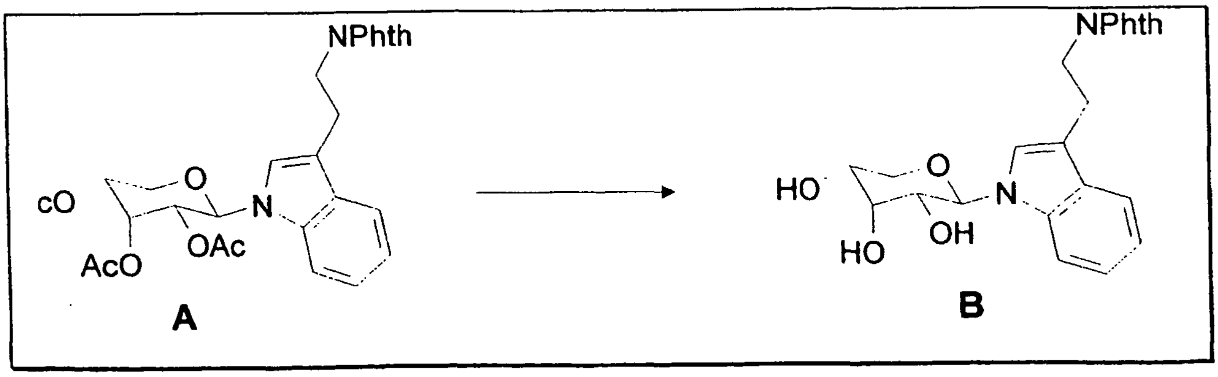

Unter Stickstoffatmosphäre wurden 44.1 g (80 mmol) **A** in 400 ml wasserfreiem Methanol gelöst. Unter Eiskühlung versetzte man mit 4.0 ml 30%iger Natriummethylatlösung und rührte dann 18 h bei RT. Der ausgefallene Niederschlag wurde abgesaugt und mit kaltem Ethanol gewaschen. Das Filtrat wurde im Vak. eingeengt. Der Rückstand wurde in Dichlormethan aufgenommen. Diese Lösung wurde mit ges. $NaHCO_3$-Lösung gewaschen, über $NaSO_4$ getrocknet und im Vak. eingeengt. Der erhaltene Rückstand wurde zusammen mit dem aus der Reaktionslösung ausgefallenen Niederschlag aus heißem Ethanol umkristallisiert. Man erhielt 22.6 g **B**, Smp. 196-198 °C. Die Mutterlauge wurde im Vakuum eingeengt und der Rückstand erneut aus Ethanol umkristallisiert. Man erhielt weitere 9.2 g **B**, Smp. 188-194 °C. Gesamtausbeute: 25.8 g **B**, d. s. 76%.

$^1$H-NMR (MeOD, 300 MHz): 3.09 (app. t, J = 7.0 Hz, 2 H, $C\underline{H}_2CH_2$NPhth), 3.64-3.98 (m, 5 H, H-4', H-5'ax, H-5'eq, $C\underline{H}_2$NPhth), 4.05 (dd, J = 3.5, 9.5 Hz, 1 H, H-2'), 4.22 (app t, J = 3.0 Hz, 1 H, H-3'), 5.65 (d, J = 9.5 Hz, 1 H, H-1'), 6.95-7.05, 7.09-7.16 (2 m, 2 x 1 H, H-5, H-6), 7.25 (s, 1 H, H-2), 7.44 (d, J = 8.0 Hz, 1 H, H-7), 7.60 (d, J = 8.0 Hz, 1 H, H-4), 7.74-7.84 (m, 4 H, Phth).

$^{13}$C-NMR ($d_6$-DMSO, 75 MHz): 23.87, 37.79 (2 t, $\underline{C}H_2\underline{C}H_2$NPhth), 64.82 (t, C-5'), 66.74 (d, C-4'), 68.41 (d, C-2'), 71.42 (d, C-3'), 81.37 (d, C-1'), 110.42 (d, C-7), 111.05 (s, C-3), 118.17, 119.21, 121.36, 122.92, 123.80 (5 d, C-2, C-4, C-5, C-6, NPhth), 127.86, 131.59 (2 s, C-3a, Phth), 134.27 (d, Phth), 136.62 (s, C-7a), 167.72 (s, C=O).

MS (ES$^-$): 457 (M+OH$^-$ +$H_2O$, 49%), 439 (M+OH$^-$, 100%), 421 (M-H$^+$, 28%)

**1-(2'-O-Benzoyl-β-D-ribo-pyranosyl)-3-(N-phthaloyl-2-aminoethyl)-indol**

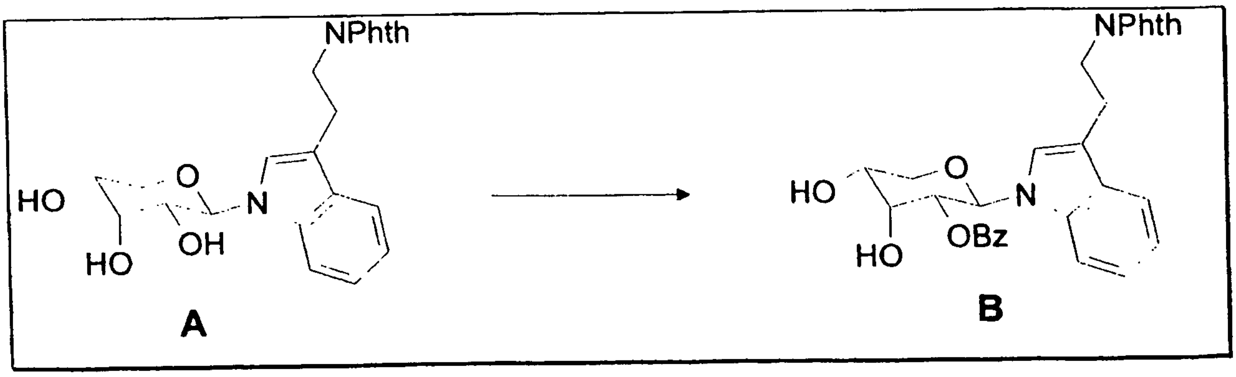

Unter Stickstoffatmoshäre wurde 10.6 g (25 mmol) **A** in 250 ml trockenem Dichlormethan aufgenommen. Man versetzt mit 305 mg DMAP (2.5 mmol) und 20 ml Pyridin. Es wurde erwärmt bis alles in Lösung war und anschließend auf -78 °C abgekühlt. Jetzt wurde 3.35 ml Benzoylchlorid (28.8 mmol) gelößt in 8 ml Dichlormethan innerhalb von 15 min zugetropft. DC-Kontrolle (EtOAc/Hexan 3:1) nach weiteren 30 min zeigte vollständige Reaktion an. Nach 45 min wurde die kalte Lösung über einen Faltenfilter direkt auf 200 ml ges. $NH_4$Cl-Lösung gegeben und der Filterrückstand wurde mit Dichlormethan gewaschen. Die organische Phase wurde einmal mit Wasser gewaschen, über $MgSO_4$ getrocknet und eingeengt. Der Rückstand wurde 2 mal mit Toluol coevaporiert und durch Flashchromatographie an 10 x 20 cm Kieselgel mit EtOAc/Hexan 3:1 gereinigt. Man erhiehlt 8.1 g **B** (64%).

$^1$H-NMR ($CDCl_3$, 300 MHz): 2.45, 2.70 (2 bs, 2 x 1 H, OH), 3.04 (t, J = 8.0 Hz, 2 H, $C\underline{H}_2CH_2$NPhth), 3.80-4.20 (m, 5 H,

H-4', H-5'ax, H-5'eq, C<u>H</u>$_2$NPhth), 4.63 (bs, 1 H, H-3'), 5.46 (dd, J = 3.5, 9.5 Hz, 1 H, H-2'), 6.03 (d, J = 9.5 Hz, 1 H, H-1'), 7.08-7.31 (m, 5 H, H-2, H-5, H-6, Bz-m-H), 7.41-7.48 (m, 1 H, H-Bz-p-H), 7.50 (d, J = 8.0 Hz, 1 H, H-7), 7.64-7.79 (m, 7 H, Phth, H-4, Bz-o-H).

$^{13}$C-NMR ($d_6$-DMSO, 75 MHz): 24.40, 38.22 (2 t, <u>C</u>H$_2$<u>C</u>H$_2$NPhth), 65.95 (t, C-5'), 66.65 (d, C-4'), 69.55 (d, C-3'), 71.87 (d, C-2'), 79.57 (d, C-1'), 109.96 (d, C-7), 113.70 (s, C-3), 119.21, 120.21, 122.11, 122.41, 123.14 (5 d, C-2, C-4, C-5, C-6, NPhth), 128.28 (d, Bz), 128.58, 128.59 (2 s, C-3a, Bz), 129.62 (d, Phth), 132.05 (s, Phth), 133.81 (Bz), 136.97 (s, C-7a), 165.12, 168.29 (2 s, C=O).

MS (ES$^-$): 525 (M-H$^+$, 12%), 421 (M-PhCO$^+$, 23%), 107 (100%).

**1-{3'-O-Benzoyl-4'O-[(4,4'-dimethoxytriphenyl)-methyl]-β-D-ribo-pyranosyl}-3-(N-phthaloyl-2-aminoethyl)-indol**

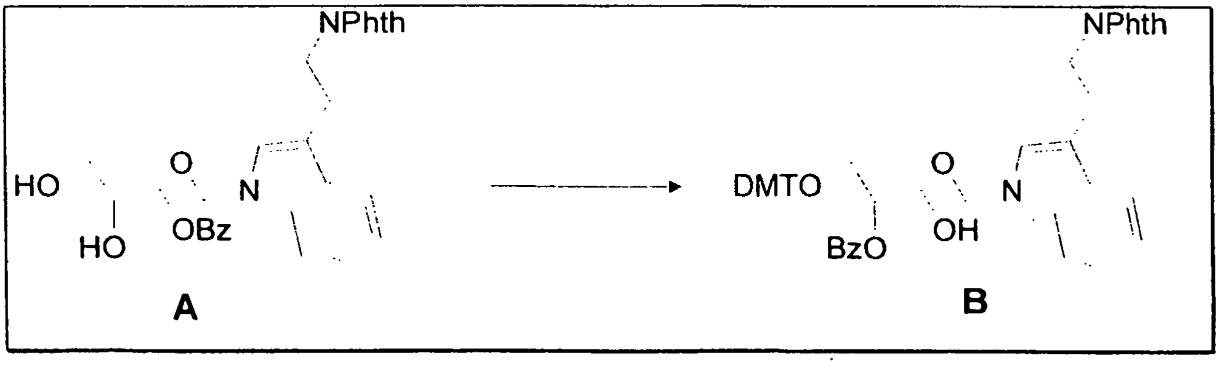

Unter Stickstoffatmoshäre wurde 8.9 g (16.9 mmol) **A** in 135 ml trockenem Dichlormethan suspendiert. Man versetzte mit 206 mg DMAP (1.68 mmol), 5.8 ml N-Ethyldiisopropylamin (33.7 mmol) und ca. 12 ml Pyridin (bis zur vollständigen Lösung). Jetzt wurde mit 34 g Molsieb 4≠ versetzt und 30 min gerührt. Nach Abkühlen auf 0°C wurde mit 11.4 g DMTCl (33.7 mmol) versetzt und nach Entfernen des Kühlbads 75 min gerührt. Dann wurden nochmals 1.94 g (0.34 eq) und nach weiteren 40 min 1.14 g (0.2 eq) und nach weiteren 65 min 1.14 g DMTCl (0.2 eq) zugegeben. Nach 4.25 h war die Reaktion beendet. Man versetzte dann mit 25.3 ml n-Propanol (20 eq), rührte noch 30 min nach und engte dann vorsichtig ein (Schaumbildung). Der Rückstand wurde in 100 ml Pyridin gelöst. Man versetzte mit 1.85 g DMAP (15.1 mmol; 0.9 eq), 13.05 ml N-Ethyldiisopropylamin (101 mmol; 6.0 eq), 71 ml n-Propanol (940 mmol; 56 eq) und 3.74 g p-Nitrophenol (26.9 mmol; 1.6 eq). Diese Mischung wurde unter Stickstoff 96 h bei 75-80 °C gerührt. Nach Abkühlen auf Raumtemperatur wurde die Mischung über Celite filtiert und eingeengt. Der Rückstand wurde durch Flashchromatographie an 9 × 17 cm Kieselgel mit Toluol/Diethylether/Triethylamin 90:10:1 gereinigt. Die produkthaltigen Fraktionen (9.25 g) wurden zunächst aus EtOAc umkristallisiert und anschließend aus Toluol/Methanol umgefällt. Man erhielt 5.86 g **B** (42%).

$^1$H-NMR (CDCl$_3$, 300 MHz): 2.64 (bs, 1 H, OH), 2.68 (dd, J = 5.0, 11.5 Hz, 1 H, H-5'eq), 2.94 (dd, J = 7.5, 16.0 Hz, 1 H, C<u>H</u>$_2$CH$_2$NPhth), 3.03 (dd, J = 8.0, 16.0 Hz, 1 H, C<u>H</u>$_2$CH$_2$NPhth), 3.67-3.74 (m, 1 H, H-5'ax), 3.69, 3.70 (2 s, 2 x 3 H, OMe), 3.85 (t, J = 7.5 Hz, 2 H, CH$_2$C<u>H</u>$_2$NPhth), 3.94 (ddd, J = 3.0, 5.0, 10.5 Hz, 1 H, H-4'), 4.03 (dd, J = 3.5, 9.0 Hz, 1 H, H-2'), 5.51 (d, J = 9.0 Hz, 1 H, H-1'), 5.86 (bs, 1 H, H-3'), 6.68-7.66 (m, 25 H), 8.19-8.30 (m, 2 H).

$^{13}$C-NMR (CDCl$_3$, 75 MHz): 24.16, 38.80 (2 t, <u>C</u>H$_2$<u>C</u>H$_2$NPhth), 55.25, 55.26 (2 q, Ome), 65.58 (t, C-5'), 68.29, 69.19, 73,83 (3 d, C-2', C-3', C-4'), 83.03 (d, C-1'), 87.31 (<u>C</u>Ar$_3$)110.03 (d, C-7), 113.37, 113.47 (2 d), 113.53 (s, C-3), 118.95, 120.20, 122.28, 122.31, 123.10, 127.07, 128.02, 128.08, 128.68 (9 d), 128.74 (s), 130.02, 130.19, 130.22 (3 d), 130.37, 131.95 (2 s), 133.40, 133.83 (2 d), 135.98, 136.14, 136.56, 145.12, 158.82, 166.76, 168.52 (7 s, C-7a, 2 COMe, 2 C=O).

**1-{2'0-(Allyloxy)(diisopropylamino)phosphino)-3'-O-Benzoyl-4'O-{(4,4'-dimethoxytriphenyl)-methyl]-β-D-ribo-pyranosyl}-3-(N-phthaloyl-2-aminoethyl)-indol**

(2 Diastereomere)

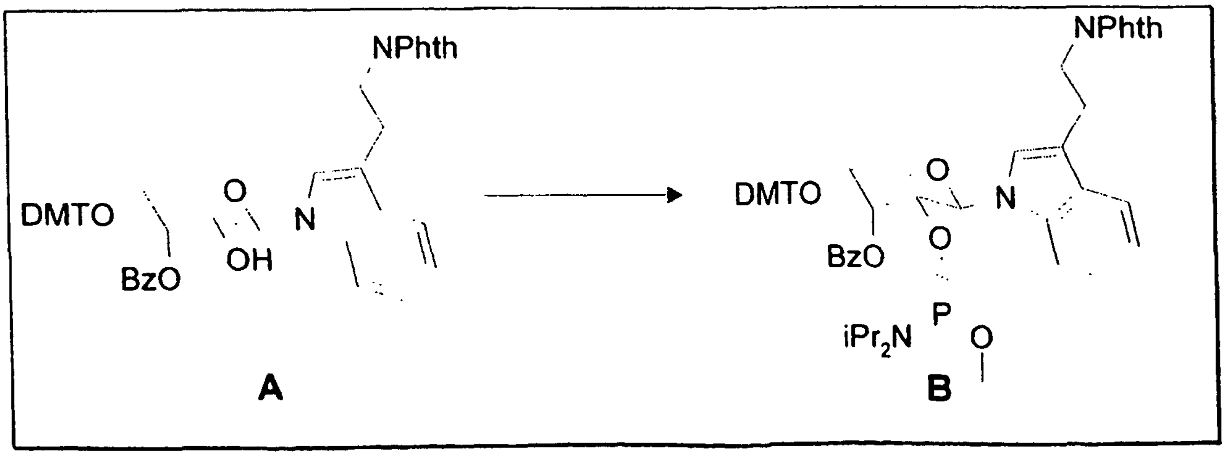

Unter Argonatmosphäre wurde 1658 mg Alkohol A (2.0 mmol) in 10 ml trockenem Dichlormethan gelöst. Man versetzte mit 1.03 ml N-Ethyldiisopropylamin (6.0 mmol) und 0.63 ml Phosphongsäuremonoallylesterdiisopropylamidchlorid (2.8 mmol) und rührte 1 h bei Raumtemperatur. Dann wurde das überschüssige Phosphorylierungsreagenz durch Zugabe von 61 µl (0.8 mmol) Isopropanol zerstört. Nach 10 min wurde im Vakuum eingeengt und der Rückstand durch Flashchromatographie an 3.3 x 21 cm Kieselgel mit Hexan/EtOAc/$NEt_3$ (75:25: 1) gereinigt. Die produkthaltigen Fraktionen wurden eingeengt, in $CCl_4$ aufgenommen und wieder eingeengt. Man erhielt 2.04 g eines fast farblosen Schaums (quant.), der so direkt zur Oligomerisierung verwendet werden kann und bei -20 °C mehrere Wochen haltbar ist. DC auf Kieselgel (EtOAc/Hexan/ $NEt_3$ 33:66:1): 0.41

$^1$H-NMR ($CDCl_3$, 300 MHz): Ausgewählte charakteristische Lagen: 2.42, 2.53 (2 dd, J = 5.0, 11.0 Hz. 2H, 2 H-5'eq), 3.76, 3.77, 3.78, 3.79 (4 s, 4 × 3 H, OMe), 5.70, 5.73 (2 d, J = 9.0 Hz, 2 H, 2 H-1'), 6.16, 6.29 (2 bs, 2 H, 2 H-3').

$^{31}$P-NMR ($CDCl_3$): 150.6, 151.0

Beispiel 3

**Lysin-basierender Linker**

Die Synthese ist im Schema 4 abgebildet und wird im folgenden im Detail beschrieben.

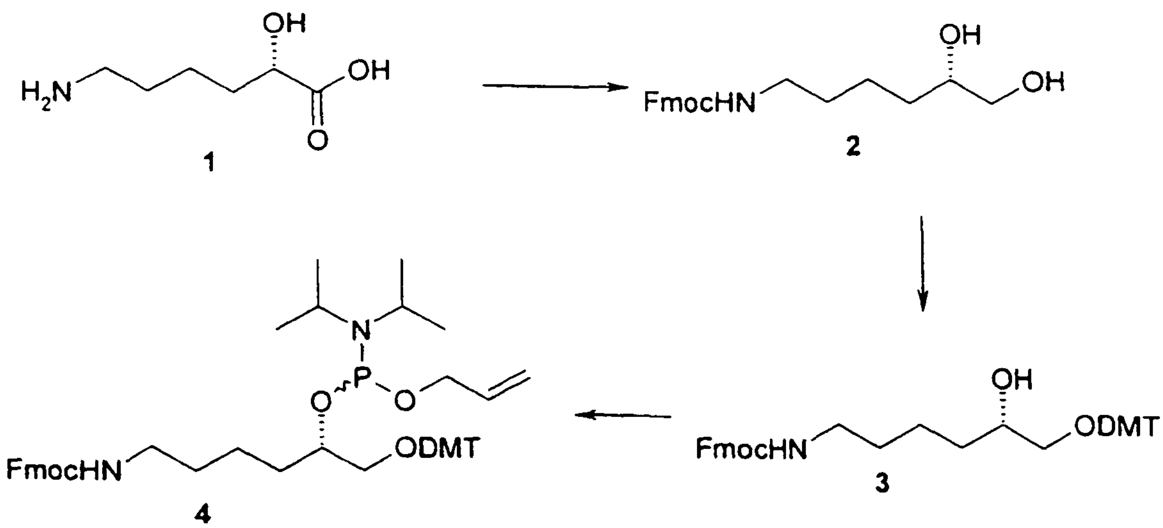

Schema 4: Synthese des Lysinlinkers

6-Amino-2(S)-hydroxyhexansäure (1) wurde nach literaturbekannter Weise durch Diazotierung und anschließende Hydrolyse aus L-Lysin hergestellt (K.-I. Aketa, Chem. Pharm Bull. 1976, 24, 621).

2-(S)-N-Fmoc-6-amino-1,2-hexandiol (**2**)

3.4 g $LiBH_4$ (156 mmol, 4 eq) werden unter Argon in 100 ml abs. THF gelöst (exotherm!). Nach Abkühlen auf ca. 30 °C werden 39.6 ml TMSCl (312 mmol, 8 eq) langsam zugetropft (Gasentwicklung!), wobei sich ein Niederschlag bildet. Anschließend werden im Argon-Gegenstrom 5.74 g 6-Amino-2(S)-hydroxyhexansäure (1) (39 mmol) portionsweise zugegeben und es wird auf 65 °C erwärmt, bis das DC (Kieselgel; i-PrOH/konz. $NH_4OH$/Wasser 7:2:1; Anfärben mit Ninhydrin) kein Edukt mehr zeigt (ca. 3 h). Unter Eiskühlung wird vorsichtig mit 120 ml Methanol versetzt (starke Gasentwicklung!). Das Lösungsmittel wird im Vakuum eingeengt, der Rückstand wird 3 mal mit je 200 ml Methanol coevaporiert und anschließend in 100 ml abs. DMF gelöst. Nach Zugabe von 16 ml Ethyldiisopropylamin (93.6 mmol. 2.4 eq) wird die Mischung auf 0°C gekühlt und portionsweise mit 12.1 g FmocCl (46.8 mmol. 1.2 eq) versetzt. Nach 15 Minuten wird das Kühlbad entfernt und bei Raumtemperatur gerührt bis das Edukt verbraucht ist (ca. 3 h; DC-Kontrolle: Kieselgel; $CHCl_3$/MeOH/HOAc/Wasser 60:30:3:5). Die Reaktionslösung wird auf 600 ml ges. $NaHCO_3$-Lösung gegeben. Der Niederschlag wird abfiltriert, mit 200 ml Wasser gewaschen und bei 50 °C am HV getrocknet bis zur Gewichtskonstanz (ca. 6 h). Man erhält 13.9 g eines farblosen Feststoffs, der aus Ethylacetat (40 ml)/n-Hexan (35 ml) umkristallisiert wird. Ausbeute: 9.05 g (65%).
$^1$H-NMR (300 MHz, $CDCl_3$): 7.68, 7.51 (2 d, J = 8.0 Hz, je 2 H, Ar-H), 7.32 (t. J = 8.0 Hz, 2 H, Ar-H), 7.23 (dt, J = 1.6, 8.0 Hz, 2 H, Ar-H), 4.92 (bs, 1 H. NH), 4.32 (d, J = 7.0 Hz, 2 H, $OCOCH_2$), 4.13 (bt, J = 7.0 Hz, 1 H, $OCOCH_2C\underline{H}$), 3.64-3.58 (m, 1 H, H-1, H-1'. H-2, H-6, H-6'), 3.54 (dd, J = 3.2, 11.0 Hz, 1 H, H-1, H-1', H-2, H-6, H-6'), 3.35 (dd, J = 7.4, 11.0 Hz, 1 H, H-1, H-1', H-2, H-6, H-6'), 3.16-3.06 (m, 2 H, H-1, H-1', H-2, H-6, H-6'), 3.0-2.0 (bs, 2 H, OH), 1.52-1.18 (m, 6 H, H-3, H-3', H-4, H-4', H-5, H-5').
2-(S)-N-Fmoc-$O^1$-DMT-6-amino-1,2-hexandiol (3) wurde nach WO 89/02439 DM-trityliert.
2-(S)-N-Fmoc-$O^1$-DMT-$O^2$-allyloxydiisopropylaminophosphinyl-6-amino-1,2-hexandiol (**4**)

Zu einer Lösung von 670 mg des Alkohols (**3**) (1.02 mmol) in 10 ml abs. Dichlormethan werden unter Argon 0.51 ml Ethyldiisopropylamin (3.0 mmol, 3 eq) und 0.33 ml Chlor-N,N-diisopropylaminoallyloxyphosphin (1.5 mmol, 1.5 eq) gegeben. Man rührt 2 h bei Raumtemperatur, zieht das Lösungsmittel im Vakuum ab und reinigt den erhaltenen Rückstand durch Flashchromatographie an 3.2 x 16 cm Kieselgel (EtOAc/iso-Hexan/$NEt_3$ 20:80:1). Man erhält 839 mg (97%) eines leicht gelblichen Öls.
DC: Kieselgel; EtOAc/iso-Hexan/$NEt_3$ 50:50:1; UV; $R_f$ = 0.77.
$^1$H-NMR (300 MHz, $CDCl_3$): 7.70-6.68 (m, 21 H, Ar-H), 4.92-4.62 (m, 1 H. NH), 4.31 (d. J = 7.0 Hz. 2H, $OCOCH_2$), 4.13 (t, J = 7.0 Hz, 1 H. $OCOCH_2C\underline{H}$), 3.98-3.40 (m, 5 H), 3.77 (2 s, je 3 H, OMe), 3.16-2.86 (m, 4 H), 2.58 (t, J = 7.0 Hz, 1 H, CHCN), 2.38 (t, 1 H, CHCN), 1.80-1.20 (m, 6 H), 1.20. 1,18, 1.17, 1.16, 1.15, 1.13, 1.08, 1.06 (8s, 12H, NMe).
$^{31}$P-NMR (300 MHz, $CDCl_3$): 149.5. 149.0 (2 s)

Beispiel 4

**Synthese eines p-RNA-Oligos der Sequenz 4'-Indollinker-A8-2' unter Verwendung von Benzimidazoliumtriflat als Kupplungsreagenz**

108 mg Indollinker-Phosphoramidit und 244 mg A-Phosphoramidit werden in Synthesizergläschen eingewogen und für 3 h im Exsikkator über KOH zusammen mit dem mit 28,1 mg CPG-Träger, beladen mit A-Baustein, gefüllten Säulchen am HV belassen. Die Phosphoramidite werden in 1 ml (Indollinker) bzw. 2,5 ml (A-Phosphoramidit) Acetonitril gelöst und einige Kügelchen von dem Molsieb zugesetzt und über KOH geschlossen im Exsikkator belassen. 200 mg Jod werden unter starkem Rühren in 50 ml Acetonitril gelöst. Nachdem alles gelöst ist (Sichtkontrolle) werden 23 ml Wasser und 4,6 ml sym-Collidin zugegeben und die Lösung einmal gut durchmischt. Zum Detritylieren wird eine 6 %ige Lösung von Dichloressigsäure in Dichlormethan eingesetzt. Das Cappingreagenz (Acetanhydrid + Base) wird wie für Oligonucleotidsynthese üblich gekauft und verwendet.

Benzimidazoliumtriflat wird aus heißem Acetonitril umkristallisiert und getrocknet. Mit den fast farblosen Kristallen wird eine 0,1 M Lösung in wasserfreiem Acetonitril als Kupplungsreagenz hergestellt. Während der Synthese bleibt diese Lösung immer klar und es kommt zu keinen Verstopfungen der Synthesizerschläuche.
Abgeänderter DNA-Kupplungscyclus am Eppendorf Ecosyn 300+ (DMT-on):

| | |
|---|---|
| Detritylierung | 7 Minuten |
| Kupplung | 1 Stunde |
| Capping | 1,5 Minuten |
| Oxidation | 1 Minute |

20 mg Tetrakis(triphenylphosphin)palladium wird in 1,5 ml Dichlormethan gelöst. 20 mg Diethylammoniumhydrogencarbonat. 20 mg Triphenylphosphin und der das Oligonucleotid tragende Glasträger zugesetzt, dicht verschlossen (Parafilm) und das Gläschen 5 h bei RT. bewegt. Dann wird der Glasträger über eine Analysennutsche abgesaugt, und mit Dichlormethan, mit Aceton und mit Wasser gewaschen.

Der Träger wird mit wäßriger 0,1molarer Natriumdiethyldithiocarbamatlösung aufgeschlämmt und 45 min bei RT. belassen. Man saugt ab, wäscht mit Wasser. Aceton. Ethanol und Dichlormethan. Der Träger wird in 1.5 ml 24 %iger Hydrazinhydratlösung suspendiert. 24-36 h lang bei 4 °C gerüttelt und mit 0,1molarem Triethylammoniumhydrogencarbonatpuffer (TEAB-Puffer) auf 7 ml verdünnt. Über eine Waters Sep-Pak Cartridge wurde hydrazinfrei gewaschen. Es wird mit 5 ml einer 80%igen Ameisensäurelösung versetzt, und nach 30 min zur Trockene einrotiert. Der Rückstand wird in 10 ml Wasser aufgenommen, mit Dichlormethan extrahiert, die wäßrige Phase eingeengt und nun HPL-chromatographiert (tR = 33 min, Gradient von Acetonitril in 0,1 M Triethylammoniumacetat-Puffer). Übliche Entsalzung (Waters Sep-Pak Cartridge) liefert das Oligonucleotid.
Ausbeute: 17,6 OD.
Substanzidentität nachgewiesen durch ESI-Massenspektroskopie:

$$M(ber) = 3082\ D,\ (M+2H)^{2+}(gef) = 1541.9\ D.$$

Beispiel 5

Herstellung von Konjugaten

1. Sequentielles Verfahren

Zuerst wird, wie in Beispiel 2 beschrieben, ein p-RNA-Oligomeres der Sequenz $A_8$, d. h. ein Octamer, auf dem Eppendorf Ecosyn D 300+ hergestellt und dann die folgenden Reagenzien ausgetauscht: 6%ige Dichloressigsäure gegen 2%ige Trichloressigsäure. Jod in Collidin gegen Jod in Pyridin, Benzimidazoltriflatlösung gegen Tetrazollösung. Nach Änderung des Syntheseprogramms wird ein DNA-Oligomeres der Sequenz GATTC nach bekannten Methoden (M. J. Gait, Oligonucleotide Synthesis, IRL Press, Oxford, UK 1984) weiter synthetisiert. Die Deallylierung, Hydrazinolyse, HPL-Chromatographie und/oder Gelelektrophorese und Entsalzung erfolgt wie für das p-RNA-Oligomere beschrieben (siehe oben) und liefert das gewünschte Konjugat.

2. Konvergentes Verfahren

Wie in Beispiel 2 beschrieben, wird ein p-RNA-Oligomeres mit der Sequenz 4'-Indollinker-$A_8$-2' hergestellt, aufgereinigt, und jodacetyliert. Ein DNA-Oligomeres der Sequenz GATTC-Thiol-Linker wird nach bekannten Methoden (M. J. Gait, Oligonucleotide Synthesis. IRL Press, Oxford, UK 1984) synthetisiert und aufgereinigt (3'-Thiol-Linker von Glen Research: Nr. 20-2933). Beim Stehenlassen der beiden Fragmente (T. Zhu et al., Bioconjug. Chem. 1994, 5, 312) in gepufferter Lösung entsteht das Konjugat, das abschließend über HPLC aufgereinigt wird.

Beispiel 6

Synthese eines einen Linker enthaltenden p-RNA-Oligonucleotids mit Linker der Formel 4' AGGCAIndT 2':

1.1 Festphasensynthese des Oligonucleotids

A, G, C, T steht für die Nucleobasen Adenin, Guanin. Cytosin und Thymin und Ind bedeutet Aminoethylindol (Indol $CH_2$-$CH_2$-$NH_2$) als Linker in Form einer Nucleobase.

Die vollautomatische Festphasensynthese wurde mit jeweils 15 μmol durchgeführt. Ein Synthesezyklus besteht aus den folgenden Schritten:

(a) Detritylierung: 5 Minuten mit 6% DCA (Dichloressigsäure) in $CH_2Cl_2$ (79 ml).
(b) Waschen mit $CH_2Cl_2$ (20 ml), Acetonitril (20 ml) und danach Spülen mit Argon;
(c) Kupplung: Waschen des Harzes mit dem Aktivator (0,5 M • Pyridin.HCl in $CH_2Cl_2$ (0,2 ml) und anschließend 30minütiges Behandeln mit Aktivator (0,76 ml) und Phosphoramidit der entsprechenden Nucleobase (0, 76 ml : 8 eq; 0,1 M in Acetonitril) im Verhältnis 1/1;
(d) Capping: 2-minütiges Behandeln mit 50% Cap A (10.5 ml) und 50% Cap B (10,5 ml) von PerSeptive Biosystems, Inc., Texas, USA (Cap A: THF, Lutidine. Acetanhydrid; Cap B: 1-Methylimidazol, THF, Pyridin);

(e) Oxidation: 1-minütiges Behandeln mit 120 ml Iodlösung (400 mg Jod in 100 ml Acetonitril, 46 ml $H_2O$ und 9,2 ml sym-Collidine); und

(f) Waschen mit Acetonitril (22 ml).

Zur Erleichterung der nachfolgenden HPLC Reinigung der Oligonucleotide wurde die letzte DMT(Dimethoxytrityl)-Gruppe nicht abgespalten. Zum Nachweis der letzten Kupplung mit den modifizierten Phorphoramiditen wurde nach der Synthese mit 1% des Harzes eine Tritylkationabsorption in UV (503 nm) durchgeführt.

1.2 Aufarbeitung des Oligonucleotids:

Die Abspaltung der Allyletherschutzgruppen erfolgte mit einer Lösung von Tetrakis(triphenylphosphin)palladium (272mg), Triphenylphosphin (272 mg) und Diethylammoniumhydrogencarbonat in $CH_2Cl_2$ (15ml) nach 5 Stunden bei RT. Die Glasträger wurden danach mit $CH_2Cl_2$ (30ml), Aceton (30ml) und Wasser (30ml) gewaschen. Um Palladiumkomplexreste zu entfernen, wurde das Harz mit einer wäßrigen 0,1 M Natriumdiethyldithiocarbamathydratlösung gespült. Die obenerwähnte Waschoperation wurde in einer umgekehrten Reihe noch einmal durchgeführt. Anschließend wurde das Harz am Hochvakuum 10 Minuten getrocknet. Der Abspaltungsschritt vom Glasträger bei gleichzeitiger Debenzoylierung wurde in 24% Hydrazinhydratlösung (6ml) bei 4°C durchgeführt. Nach HPLC-Kontrolle an RP 18 (18-25 Stunden) wurde das Oligonucleotid "Trityl ON" mittels einer aktivierten (Acetonitril, 20 ml) Waters Sep-Pak Cartridge vom Hydrazin befreit. Das Hydrazin wurde mit TEAB, 0,1 M (30ml) gewaschen. Das Oligonucleotid wurde dann mit Acetonitril/TEAB, 0,1M (10ml) eluiert. Anschließend wurde mittels HPLC zur Abtrennung von Abbruchsequenzen gereinigt und die DMT-Entschützung (30 ml 80%ig wäßrige Ameisensäure) durchgeführt. Abschließende Entsalzung (über Sep-Pak Kartusche, mit TEAB Puffer 0,1M/Acetonitril: 1/1) lieferte das reine Oligonucleotid.

Beispiel 7

Jodacetylierung von *p*-RNA mit N-(Jodacetyloxy)-succinimid

*p*-RNA-Sequenz : 4' AGGCAIndT 2' $M_w$ = 2266,56 g/mol, hergestellt gemäß Beispiel 1.

1 eq. der *p*-RNA wurde in einer 0,1 molaren Natriumhydrogencarbonatlösung (pH 8,4) gelöst (1 ml pro 350 nmol) und mit einer Lösung von N-(Jodacetyloxy)-succinimid in DMSO versetzt (40 µl pro mg). Man dunkelt den Ansatz mit Aluminiumfolie ab und ließ ihn bei Raumtemperatur für 30-90 Minuten stehen.

Der Fortgang der Reaktion wurde mittels analytischer HPLC verfolgt. Die Standardbedingungen waren:

Puffer A : 0,1 molarer Triethylammoniumacetat-Puffer in Wasser
Puffer B : 0,1 molarer Triethylammoniumacetat-Puffer in Wasser:Acetonitril 1:4
Gradient : von 10% B startend auf 50% B in 40 Minuten
Säulenmaterial: 10 µM LiChrosphere® 100 RP-18 von Merck Darmstadt GmbH; 250 × 4 mm Retentionszeit der Edukte: 18,4 Minuten
Retentionszeit der Produkte in diesem Falle : 23,1 Minuten

Nach beendeter Reaktion wurde der Ansatz mit Wasser auf das vierfache Volumen verdünnt. Man aktivierte eine Waters Sep-Pak-Kartusche RP-18 (ab 15 OD 2 g Füllung) mit 2 x 10 ml Acetonitril und 2 x 10 ml Wasser, trug das Oligonucleotid auf, ließ einsinken, wusch das Reaktionsgefäß mit 2 x 10 ml Wasser, wusch mit 3 x 10 ml Wasser nach, um Salz und Reagenz zu entfernen, und eluierte zuerst mit 5 x 1 ml 50:1 Wasser : Acetonitril und anschließend mit 1: 1. Das Produkt eluierte in den 1:1-Fraktionen in sehr guter Reinheit. Die Fraktionen wurden in der Kälte und im Dunkeln eingeengt, vereinigt, und wieder eingeengt.

Die Ausbeuten wurden mittels UV-Absorptionspektrometrie bei 260 nm bestimmt.

Massenspektrometrie :

Sequenz : 4' AGGCAInd($CH_2CH_2NHCOCH_2$-I)T 2' berechnete Masse : 2434.50 g/mol gefundene Masse $MH_2^{2+}$: 1217.9 g/mol = 2433

Beispiel 8

Konjugation von *p*-RNA an ein definiertes Peptid (CYSKVG)

Die jodacetylierte *p*-RNA ($M_w$ = 2434.50 g/mol) wurde in einem Puffersystem gelöst (1000µl pro 114 nmol) und dann mit einer Lösung des Peptides in Puffer versetzt (2 moleq. CYSKVG-Peptid; $M_w$ = 655.773 g/mol; 228 nmol in 20 µl Puffer).
Puffersystem : Borax/HCl-Puffer der Firma Riedel-de Haën, pH 8.0, wurde im Verhältnis 1:1 mit einer 10 millimolaren Lösung von EDTA-Dinatriumsalz in Wasser gemischt und auf pH 6.3 mit HCl eingestellt. Man erhielt dadurch eine Lösung, die 5 mM $Na_2EDTA$ enthält.
Man beließ den Ansatz bis zum vollständigen Umsatz bei Raumtemperatur im Dunkeln. Die Reaktion wurde mittels HPLC-Analytik verfolgt.
Die Standardbedingungen waren :

Puffer A : 0,1 molarer Triethylammoniumacetat-Puffer in Wasser
Puffer B : 0,1 molarer Triethylammoniumacetat-Puffer in Wasser:Acetonitril 1:4
Gradient: von 10% B startend auf 50% B in 40 Minuten
Säulenmaterial : 10 µM LiChrosphere® 100 RP-18 von Merck Darmstadt GmbH; 250 x 4
Retentionszeit des Eduktes : 17,6 Minuten
Retentionszeit des Produktes : 15,5 Minuten
Nach beendeter Reaktion wurde der Ansatz direkt mittels RP-HPLC gereinigt.

(Standardbedingungen siehe oben).

Die Fraktionen wurden in der Kälte und im Dunkeln eingeengt, vereinigt, und wieder eingeengt. Man nahm in Wasser auf und entsalzte. Man aktivierte eine Waters Sep-Pak-Kartusche RP-18 (ab 15 OD 2 g Füllung) mit 2 x 10 ml Acetonitril und 2 x 10 ml Wasser, trug das Oligo auf, ließ einsinken, wusch das Reaktionsgefäß mit 2 x 10 ml Wasser, wusch mit 3 x 10 ml Wasser nach, um das Salz zu entfernen, und eluierte mit Wasser : Acetonitril 1:1. Die Produkt-Fraktionen wurden eingeengt, vereinigt, und wieder eingeengt.
Die Ausbeuten wurden mittels UV-Absorptionspektrometrie bei 260 nm bestimmt. Sie erreichten 70-95% der Theorie.

Massenspektrometrie :

Sequenz : 4' AGGCAInd($CH_2CH_2NHCOCH_2$-CYSKVG)T 2' berechnete Masse $MH_2^{2+}$: 2962,36 g/mol gefundene Masse $MH_2^{2+}$: 1482,0 g/mol

Beispiel 9

Konjugation von p-RNA an eine Peptidbibliothek

Die jodacetylierte p-RNA ($M_w$ = 2434,50 g/mol) wurde in einem Puffersystem gelöst (1300 µl pro 832 nmol) und dann mit einer Lösung der Peptidbibliothek (CKR-XX-OH: X = Arg, Asn, Glu. His. Leu, Lys, Phe, Ser, Trp, Tyr) in Puffer versetzt (8 moleq.; mittlere Molekülmasse $M_m$ = 677,82 g/mol; 4,5 mg = 6.66 µmol in 200 µl Puffer).
Puffersystem : Borax/HCl-Puffer der Firma Riedel-de Haën, pH 8,0, wurde im Verhältnis 1:1 mit einer 10 millimolaren Lösung von EDTA-Dinatriumsalz in Wasser gemischt und auf pH 6,6 mit HCl eingestellt. Man erhielt dadurch eine Lösung, die 5 mM $Na_2EDTA$ enthielt.
Man beließ den Ansatz bis zum vollständigen Umsatz bei Raumtemperatur im Dunkeln. Die Reaktion wurde mittels HPLC-Analytik verfolgt. In diesem Fall war das Edukt nach 70 Stunden verschwunden.
Die Standardbedingungen der analytischen HPLC sind :

Puffer A : 0,1 molarer Triethylammoniumacetat-Puffer in Wasser
Puffer B : 0,1 molarer Triethylammoniumacetat-Puffer in Wasser:Acetonitril 1:4
Gradient : von 10% B startend auf 50% B in 40 Minuten
Säulenmaterial : 10 µM LiChrosphere® 100 RP-18 von Merck; 250 x 4
Retentionszeit des Eduktes : 18,8 Minuten

Retentionszeit des Produktes : mehrere Peaks von 13,9-36.2 Minuten

Nach beendeter Reaktion wurde der Ansatz mit Wasser auf das vierfache Volumen verdünnt. Man aktivierte eine Waters Sep-Pak-Kartusche RP-18 (ab 15 OD 2 g Füllung) mit 3 x 10 ml Acetonitril und 3 x 10 ml Wasser, trug das Oligonucleotid auf, ließ einsinken, wusch das Reaktionsgefäß mit 2 x 10 ml Wasser nach, wusch die Kartusche mit 3 $\times$ 10 ml Wasser nach, um Salz und überschüssiges Peptid zu entfernen, und eluierte mit 1:1 Wasser : Acetonitril, bis UV-spektroskopisch kein Produkt mehr eluierte. Die Fraktionen wurden in der Kälte und im Dunkeln eingeengt, vereinigt, und wieder eingeengt.

## Patentansprüche

1. Linker-Nucleosid der Formel (I),

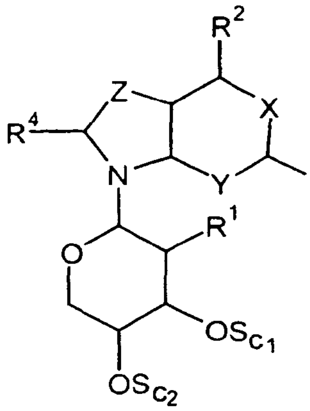

(I),

worin
$R^1$ gleich H, OH. Hal mit Hal gleich Br oder Cl,
$R^2$, $R^3$ und $R^4$ unabhängig voneinander, gleich oder verschieden, jeweils H oder $(C_nH_{2n})NR^{10}R^{11}$ mit $R^{10}R^{11}$ verbunden über einen Rest der Formel

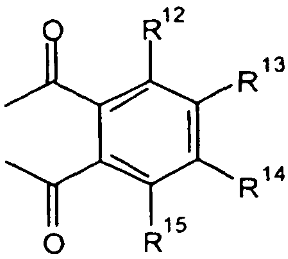

(V),

worin
$R^{12}$, $R^{13}$, $R^{14}$ und $R^{15}$ unabhängig voneinander, gleich oder verschieden, jeweils H, $C_nH_{2n+1}$, oder $C_nH_{2n-1}$ oder $OR^7$, wobei $R^7$ gleich H, $C_nH_{2n+1}$ oder $C_nH_{2n-1}$, $-C(O)R^8$ mit $R^8$ gleich ein linearer oder verzweigter Alkyl- oder Aryl-Rest, der auch weiter substituiert sein kann, mit n gleich eine ganze Zahl von 1-12,
X, Y und Z unabhängig voneinander, gleich oder verschieden, jeweils =N-, $=C(R^9)$- oder $-N(R^{9'})$- mit $R^9$ und $R^{9'}$

unabhängig voneinander, gleich oder verschieden, jeweils H oder $C_nH_{2n+1}$ oder $(C_nH_{2n})NR^{10}R^{11}$ mit den oben genannten Bedeutungen, bedeutet, oder ein Linker-Nucleosid (I) mit einem Pentopyranosyl-Nucleosidrest, wobei die Reste $R^2$, $R^3$, $R^4$ und die Gruppen X, Y und Z so ausgewählt sind, dass man Linker-Nucleoside ausgewählt aus der Gruppe Pentopyranosyl -purin, -2,6-diaminopurin, -6-purinthiol, -adenosin, - guanosin, -isoguanosin, -6-thioguanosin, -xanthin, -hypoxanthin, -indol, -tryptamin, -N-phthaloyltryptamin, -coffein, -theobromin, -theophyllin und -benzotriazol erhält,
und
$S_{c1}$ und $S_{c2}$ unabhängig voneinander, gleich oder verschieden, jeweils H oder eine Schutzgruppe ausgewählt aus einer Acyl-, Benzoyl-, Trityl-, 4,4'-Dimethoxytrityl-(DMT)- oder Allyloxycarbonylgruppe oder ein Phosphoester(III), Phosphoester (V), Thiophosphat (V), Phosphonat oder Phosphoramidit ist,
oder der Formel (III)

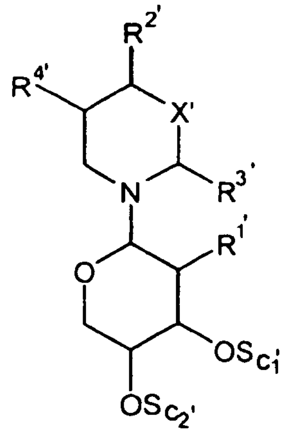

(III),

worin
$R^{1'}$ gleich H, OH,Hal mit Hal gleich Br oder Cl,
$R^{2'}$,$R^{3'}$ und $R^{4'}$ unabhängig voneinander, gleich oder verschieden, jeweils H, oder $(C_nH_{2n})NR^{10'}R^{11'}$, wobei $R^{10'}$, $R^{11'}$, unabhängig voneinander die oben genannte Bedeutung von $R^{10}$ bzw. $R^{11}$ hat und
X' jeweils =N-. =C($R^9$)- oder -N($R^{9'}$)- bedeutet, wobei $R^9$ und $R^{9'}$ unabhängig voneinander die oben genannte Bedeutung von $R^9$ bzw. $R^{9'}$ haben, oder ein Linker-Nucleosid (III) mit einem Pentopyranosyl-Nucleosidrest, wobei die Reste $R^{2'}$, $R^{3'}$, $R^{4'}$ und die Gruppe X so ausgewählt sind, dass man Linker-Nucleoside ausgewählt aus der Gruppe Pentopyranosyl -pyridin, -pyrimidin, -thymidin, -cytosin, -isocytosin und-uracil erhält,
und $S_{c1}$ bzw. $S_{c2}$. die oben genannte Bedeutung von $S_{c1}$ bzw. $S_{c2}$ haben
und worin zumindest einer der Reste $R^2$, $R^3$, $R^4$ in Formel (1) und zumindest einer der Reste $R^{2'}$, $R^{3'}$, $R^{4'}$ in Fonnel (III) ein Phthalimidoalkylrest der Formel $(C_nH_{2n})NR^{10}R^{11}$ oder einen Allyloyrest darstellt.

2. Linker-Nucleosid nach Anspruch 1, **dadurch gekennzeichnet dass** das Pentopyranosyl-Nucleosid ein Ribo- Arabino-, Lyxo- und/oder Xylo-pyranosyl-Nucleosid ist.

3. Linker-Nucleosid nach einem der Ansprüche 1 oder 2, **dadurch gekennzeichnet, daß** der Pentopyranosyl-Teil des Pentopyranosly-Nucleosids D- oder L-konfiguriert ist.

4. Linker-Nucleosid nach einem der Ansprüche 1-3, **dadurch gekennzeichnet, daß** $R^2$, $R^3$, $R^4$, $R^{2'}$, $R^{3'}$ und/oder $R^{4'}$ ein 2-Phthalimidoethyl-Rest bedeutet.

5. Verfahren zur Herstellung eines Linkers gemäß Formel (III), worin $R^{4'}$ $(C_nH_{2n})NR^{10'}R^{11'}$ bedeutet und $R^{10'}R^{11'}$ über einen Rest der Formel (V) mit der in Anspruch 1 bezeichneten Bedeutung verbunden ist, **dadurch gekennzeichnet, dass**

(a) eine Verbindung der Formel (III) mit $R^{4'}$ gleich $(C_nH_{2n})SO_{c3}$ oder $(C_nH_{2n})Hal$, worin n die oben genannte

Bedeutung hat, $S_{c3}$ eine Schutzgruppe und Hal Chlor oder Brom bedeutet,
mit einem Azid umgesetzt wird,
(b) das Reaktionsprodukt aus (a) reduziert wird,
(c) das Reaktionsprodukt aus (b) mit einem entsprechenden Phthalimid umgesetzt wird,
(d) das Reaktionsprodukt aus (c) mit einer entsprechenden geschützten Pentose umgesetzt wird, und
(e) gegebenenfalls die Schutzgruppen abgespalten werden.

**6.** Verfahren zur Herstellung eines Linkers gemäß Formel (I) worin X und Y unabhängig voneinander, gleich oder verschieden, jeweils $=C(R^{16})$- mit $R^{16}$ gleich H oder $C_nH_{2n}$ und Z $=C(R^{16})$- mit $R^{16}$ gleich $(C_nH_{2n})NR^{10}R^{11}$ mit der in Anspruch 1 bezeichneten Bedeutung, **dadurch gekennzeichnet, dass**

(a) das entsprechende Indol in mit einer Pentose zum Nucleosidtriol umgesetztwird,
(b) die Hydroxylgruppen des Pentose-Teils des Produktes aus (a) geschützt werden,
(c) das Produkt aus (b) oxidiert wird, und
(d) gegebenenfalls die Hydroxyl-Schutzgruppen des Pentose-Teils des Produktes aus (c) abgespalten werden.

**7.** Verfahren nach einem der Ansprüche 5 oder 6, **dadurch gekennzeichnet, dass** das 4'-geschützte Pentopyranosyl-nucleosid in einem weiteren Schritt phosphityliert oder an eine feste Phase gebunden wird.

**8.** Verfahren zur Herstellung einer Nucleinsäure, **dadurch gekennzeichnet, dass**

(a) in einem ersten Schritt ein geschütztes Nucleosid oder ein geschützter Linker gemäß einem der Ansprüche 1 - 4 an eine feste Phase gebunden wird und
(b) in einem zweiten Schritt das gemäß Schritt (a) an eine feste Phase gebundene 3', 4'-geschützte Nucleosid um ein phosphityliertes 3', 4'- geschütztes Nucleosid verlängert wird, anschließend pxidiert wird, und
(c) Schritt (b) solange mit gleichen oder unterschiedlichen Nucleosiden bzw. Linker-Nucleosiden wiederholt wird, bis die gewünschte Nucleinsäure vorliegt, wobei die Nucleinsäure mindestens ein genanntes Linker-Nucleosid enthält.

**9.** Verfahren nach Anspruch 8, **dadurch gekennzeichnet, dass** in Schritt (a) und/oder Schritt (b) auch Pentofuranosyl-nucleoside neben Pentopyranosyl-nucleosiden eingebaut werden.

**10.** Verfahren nach Anspruch 8 oder 9, **dadurch gekennzeichnet, dass** in einem weiteren Schritt (d) die Schutzgruppe un die gebildete Nucleinsäure von der festen Phase abgespalten wird.

**11.** Verfahren nach Anspruch 10, **dadurch gekennzeichnet, dass** die Abspaltung durch Hydrazinolyse erfolgt.

**12.** Verfahren nach einem der Ansprüche 5 bis 11, **dadurch gekennzeichnet, dass** in einem weiteren Schritt ein Allyloxy-Linker der Formel

$$S_{c4}NH(C_nH_{2n})CH(OPS_{c5}S_{c6})C_nH_{2n}S_{c7} \qquad (VI)$$

worin Sc4 und Sc7 unabhängig voneinander, gleich oder verschieden, jeweils eine Schutzgruppe.
Sc5 und Sc6 unabhängig voneinander, gleich oder verschieden, jeweils eine Allyloxy- und/oder Diisopropylamino-Gruppe und n wie in Anspruch 1 bezeichnet, bedeuten, eingaubt wird.

**13.** Nucleinsäure enthaliend mindestens ein Linker- Nucleosid gemäß einem der Ansrprüche 1 - 4 und mindestens einen Allyloxy-Linker gemäß Anspruch 12.

**14.** Konjugat enthaltend ein Linker-Nucleosid gemäß einem der Ansprüche 1-4 und/oder eine Nucleinsäure gemäß Anspruch 13 und ein Biomolekül.

**15.** Konjugat nach Anspruch 14, **dadurch gekennzeichnet, dass** das Biomolekül ein Peptid, Peptoid, Protein, Zellbestandteil, Filamentbestandteil oder eine Nucleinsäure oder ein Derivat davon ist.

**16.** Träger, **dadurch gekennzeichnet, dass** daran mindestens ein Linker-Nucleosid gemäß einem der Ansprüche 1-4, mindestens eine Nucleinsäure gemäß Anspruch 13 und/oder mindestens ein Konjugat gemäß Anspruch 14 oder 15 immobilisiert ist.

**17.** Diagnostikum enthaltend ein Linker-Nucleosid gemäß einem der Ansprüche 1-4, eine Nucleinsäure gemäß Anspruch 13 und/oder ein Konjugat gemäß Anspruch 14 oder 15.

**18.** Verwendung eines Linker-Nucleosids gemäß einem der Ansprüche 1-4, einer Nucleinsäure gemäß Anspruch 13, eines Konjugates gemäß Anspruch 14 oder 15 und/oder eines Träger gemäß Anspruch 16 zur Herstellung eines Arzneimittels, Diagnostikums und/oder elektronischen Bauteils.

**19.** Verwendung eines Linker-Nucleosids gemäß einem der Ansprüche 1-4, einer Nucleinsäure gemäß Anspruch 13, eines Konjugates gemäß Anspruch 14 oder 15 und/oder eines Träger gemäß Anspruch 16 in einem Paarungs- und/ oder Testsystem.

## Claims

**1.** Linker nucleoside of the formula (I),

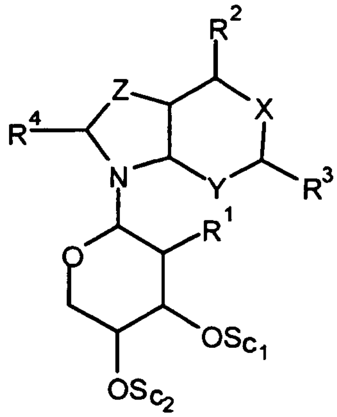

(I),

in which
$R^1$ is equal to H, OH, Hal where Hal is equal to Br or Cl,
$R^2$, $R^3$ and $R^4$ independently of one another, identically or differently, in each case are H or $(C_nH_{2n})NR^{10}R^{11}$ where $R^{10}R^{11}$ is linked via a radical of the formula

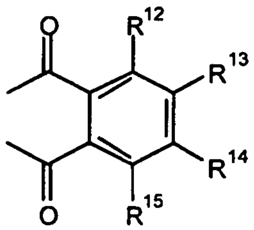

(V),

in which
$R^{12}$, $R^{13}$, $R^{14}$ and $R^{15}$ independently of one another, identically or differently, in each case are H, $C_nH_{2n+1}$ or $C_nH_{2n-1}$ or $OR^7$, where $R^7$ is equal to H, $C_nH_{2n+1}$ or $C_nH_{2n-1}$, $-C(O)R^8$ where $R^8$ is equal to a linear or branched alkyl or aryl radical, which may be further substituted, where n is equal to an integer from 1-12,
X, Y and Z independently of one another, identically or differently, in each case are =N-, $=C(R^9)-$ or $-N(R^{9'})-$ where $R^9$ and $R^{9'}$ independently of one another, identically or differently, in each case are H or $C_nH_{2n+1}$ or $(C_nH_{2n})NR^{10}R^{11}$ having the abovementioned meanings, or a linker nucleoside (I) having a pentopyranosylnucleoside radical, where

the radicals $R^2$, $R^3$, $R^4$ and the groups X, Y and Z are selected such that linker nucleosides selected from the group of pentopyranosyl-purine, -2,6-diaminopurine, -6-purinethiol, -adenosine, -guanosine, -isoguanosine, -6-thioguanosine, -xanthine, -hypoxanthine, -indole, -tryptamine, -N-phthaloyltryptamine, -caffeine, -theobromine, -theophylline or benzotriazole are obtained,
and
$S_{c1}$ and $S_{c2}$ independently of one another, identically or differently, in each case are H or a protective group selected from an acyl, benzoyl, trityl, 4, 4'-dimethoxytrityl (DMT) group, or allyloxycarbonyl group, or are a phosphoester(III), phosphoester(V), thiophosphate(V), phosphonate or phosphoramidite,
or of the formula (III)

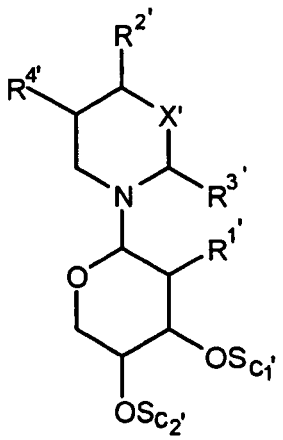

(III),

in which
$R^{1'}$ is equal to H, OH or Hal where Hal is equal to Br or Cl,
$R^{2'}$,$R^{3'}$ and $R^{4'}$ independently of one another, identically or differently, in each case are H or $(C_nH_{2n})NR^{10'}R^{11'}$, where $R^{10'}$, $R^{11'}$, independently of one another has the abovementioned meaning of $R^{10}$ or $R^{11}$,
X' in each case is =N-, =C($R^9$)- or -N($R^{9'}$)-, where $R^9$ and $R^{9'}$ independently of one another have the abovementioned meaning of $R^9$ and $R^{9'}$, or a linker nucleoside (III) having a pentopyranosylnucleoside radical, where the radicals $R^{2'}$, $R^{3'}$, $R^{4'}$ and the group X are selected such that linker nucleosides selected from the group of pentopyranosyl-pyridine, -pyrimidine, -thymidine, -cytosine, -isocytosine and -uracil are obtained,
and $S_{c1'}$ and $S_{c2'}$ have the abovementioned meaning of $S_{c1}$ and $S_{c2}$.
and in which at least one of the radicals $R^2$, $R^3$, $R^4$ in the formula (I) and at least one of the radicals $R^{2'}$, $R^{3'}$, $R^{4'}$ in the formula (III) constitute a phthalimidoalkyl radical of the formula $(C_nH_{2n})NR^{10}R^{11}$ or an allyloxy radical.

2. Linker nucleoside according to Claim 1, **characterized in that** the pentopyranosylnucleoside is a ribo-, arabino-, lyxo- and/or xylopyranosylnucleoside.

3. Linker nucleoside according to one of Claims 1 or 2, **characterized in that** the pentopyranosyl moiety of the pentapyranosylnucleoside is in the D or L configuration.

4. Linker nucleoside according to one of Claims 1-3, **characterized in that** $R^2$, $R^3$, $R^4$, $R^{2'}$, $R^{3'}$ and/or $R^{4'}$ is a 2-phthalimidoethyl radical.

5. Process for the preparation of a linker according to formula (III), in which $R^{4'}$ is $(C_nH_{2n})NR^{10'}R^{11'}$ and $R^{10'}R^{11'}$ is linked to the meaning designated in Claim 1 via a radical of the formula (V), **characterized in that**

(a) a compound of the formula (III) where $R^{4'}$ is equal to $(C_nH_{2n})OS_{c3}$ or $(C_nH_{2n})Hal$, in which n has the abovementioned meaning, $S_{c3}$ is a protective group, and Hal is chlorine or bromine,
is reacted with an azide,
(b) the reaction product from (a) is reduced,
(c) the reaction product from (b) is reacted with an appropriate phthalimide,

(d) the reaction product from (c) is reacted with an appropriate protected pentose, and
(e) if appropriate, the protective groups are removed.

**6.** Process for the preparation of a linker according to formula (I), in which X and Y independently of one another, identically or differently, in each case are =C($R^{16}$)- where $R^{16}$ is H or $C_nH_{2n}$ and Z is =C($R^{16}$)- where $R^{16}$ is equal to $(C_nH_{2n})NR^{10}R^{11}$ having the meaning designated in Claim 1, **characterized in that**

(a) the corresponding indoline is reacted with a pentose to give the nucleoside triol,
(b) the hydroxyl groups of the pentose moiety of the products from (a) are protected,
(c) the product from (b) is oxidized, and
(d) if appropriate, the hydroxyl protective groups of the pentose moiety of the product from (c) are removed.

**7.** Process according to either of Claims and 6, **characterized in that** the 4'-protected pentopyranosylnucleoside is phosphitylated or bonded to a solid phase in a further step,

**8.** Process for the preparation of a nucleic acid, **characterized in that**

(a) in a first step a protected nucleoside or a protected linker according to one of Claims 1-4 is bonded to a solid phase and
(b) in a second step the 3'-, 4'-protected nucleoside bonded to a solid phase according to step (a) is lengthened by a phosphitylated 3'-, 4'-protected nucleoside, then oxidized, and
(c) step (b) is repeated using identical or different nucleosides or linker nucleosides until the desired nucleic acid is present, the nucleic acid containing at least one mentioned linker nucleoside.

**9.** Process according to Claim 8, **characterized in that** pentafuranosylnucleosides in addition to pentopyranosylnucleosides are also incorporated in step (a) and/or step (b).

**10.** Process according to Claim 8 or 9, **characterized in that** a further step (d) the protective group and the nucleic acid formed is removed from the solid phase.

**11.** Process according to Claim 10, **characterized in that** the removal is effected by means of hydrazinolysis.

**12.** Process according to one of Claims 5 to 11, **characterized in that** in a further step an allyloxy linker of the formula

$$S_{c4}NH(C_nH_{2n})CH(OPS_{c5}S_{c6})C_nH_{2n}S_{c7} \qquad (VI)$$

in which $S_{c4}$ and $S_{c7}$ independently of one another, identically or differently, in each case are a protective group, $S_{c5}$ and $S_{c6}$ independently of one another, identically or differently, in each case are an allyloxy and/or diisopropylamino group and n is as designated in Claim 1,
is incorporated.

**13.** Nucleic acid comprising at least one linker nucleoside according to one of Claims 1-4 and at least one allyloxy linker according to Claim 12.

**14.** Conjugate comprising a linker nucleoside according to one of Claims 1-4, and/or a nucleic acid according to Claim 13 and/or a biomolecule.

**15.** Conjugate according to Claim 14, **characterized in that** the biomolecule is a peptide, peptoid, protein, cell constituent, filament constituent, or a nucleic acid, and derivatives thereof.

**16.** Carrier, **characterized in that** at least one linker nucleoside according to one of Claims 1-4, at least one nucleic acid according to Claim 13 and/or at least one conjugate according to Claim 14 or 15 is immobilized thereon.

**17.** Diagnostic comprising a linker nucleoside according to one of Claims 1-4, a nucleic acid according to Claim 13 and/or a conjugate according to Claim 14 or 15.

**18.** Use of a linker nucleoside according to one of Claims 1-4, of a nucleic acid according to Claim 13, of a conjugate according to Claim 14 or 15 and/or of a carrier according to Claim 16 for the production of a pharmaceutical, diagnostic

and/or electronic component.

**19.** Use of a linker nucleoside according to one of Claims 1-4, of a nucleic acid according to Claim 13, of a conjugate according to Claim 14 or 15 and/or of a carrier according to Claim 16 in a pairing and/or test system.

**Revendications**

**1.** Lieur nucléoside de formule (I),

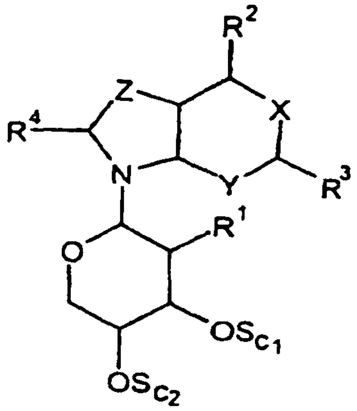

(I),

dans laquelle

$R^1$ représente H, OH, Hal, où Hal est Br ou Cl,
$R^2$ , $R^3$ et $R^4$, indépendamment les uns des autres, identiques ou différents, chacun représente H ou $(C_nH_{2n})$ $NR^{10}R^{11}$ avec $NR^{10}R^{11}$ lié par l'intermédiaire d'un reste de formule

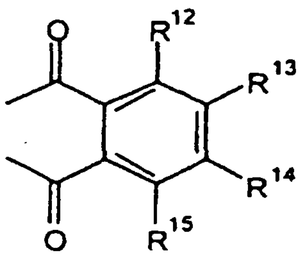

(V),

dans laquelle

$R^{12}$, $R^{13}$, $R^{14}$ et $R^{15}$, indépendamment les uns des autres, identiques ou différents, chacun représente H, $C_nH_{2n+1}$ ou $C_nH_{2n-1}$ ou $OR^7$, $R^7$ représente H, $C_nH_{2n+1}$ ou $C_nH_{2n-1}$, C (O) $R^8$, où $R^8$ représente un reste alkyle linéaire ou ramifié ou aryle qui peut aussi être substitué davantage, n étant un entier de 1 à 12,
X, Y et Z, indépendamment les uns des autres, identiques ou différents, chacun représente =N-, =C ($R^9$) - ou -N($R^{9'}$)-, où $R^9$ et $R^{9'}$ , indépendamment l'un de l'autre, identiques ou différents, chacun représente H ou $C_nH_{2n+1}$ ou $(C_nH_{2n})$ $NR^{10}R^{11}$ ayant les significations données ci-dessus, ou un lieur nucléoside (I) avec un reste nucléoside pentopyranosyle, les restes $R^2$, $R^3$ $R^4$ et les groupes X, Y et Z sont choisis de façon telle que les lieurs nucléosides soient pris dans le groupe comprenant pentopyranosyle -purine, -2,6-diaminopurine, -6-purinethiol,

-adénosine, -guanosine, -isoguanosine, -6-thioguanosine, -xanthine, -hypoxanthine, -indole, -tryptamine, -N-phtaloyltryptamine, -cofféine, -théobromine, -théophylline et -benzotriazole,

et

$S_{c1}$ et $S_{c2}$, indépendamment l'un de l'autre, identiques ou différents, chacun représente H ou un groupe protecteur pris parmi les groupes acyl-, benzoyl-, trityl-, 4,4'-diméthoxytrityl-(DMT)-ou allyloxycarbonyle ou un phosphoester(III), un phosphoester (V), un thiophosphate (V), un phosphonate ou une phosphoramidite,

ou de formule (III)

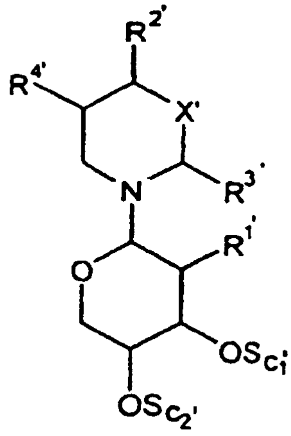

**(III),**

où

$R^{1'}$ représente H, OH, Hal, où Hal est Br ou Cl,
$R^{2'}$ , $R^{3'}$ et $R^{4'}$, indépendamment les uns des autres, identiques ou différents, chacun représente H ou $(C_nH_{2n})$ $NR^{10'}R^{11'}$, où $R^{10'}$, $R^{11'}$ , indépendamment l'un de l'autre, possèdent les significations de $R^{10}$ ou $R^{11}$ données ci-dessus et
X' représente à chaque fois =N-, =C ($R^9$) - ou -N($R^{9'}$)-, $R^9$ et $R^{9'}$, indépendamment l'un de l'autre, possèdent la signification donnée ci-dessus pour $R^9$ ou $R^{9'}$, ou un lieur nucléoside (III) avec un reste nucléoside pentopyranosyle, les restes $R^{2'}$, $R^{3'}$ , $R^{4'}$ et le groupe X sont choisis de façon telle qu'on obtienne des lieurs nucléosides choisis dans le groupe comprenant pentopyranosyle -pyridine, -pyrimidine, -thymidine, -cytosine, -isocytosine et -uracile,

et

$S_{c1}$, ou $S_{c2}$, possèdent la signification donnée ci-dessus pour $S_{c1}$ et $S_{c2}$,

et où au moins un des restes $R^2$, $R^3$, $R^4$ dans la formule (I) et au moins un des restes $R^{2'}$, $R^{3'}$, $R^{4'}$ dans la formule (III) représente un reste phtalimidoalkyle de formule $(C_nH_{2n})$ $NR^{10}R^{11}$, ou un reste allyloxy.

2. Lieur nucléoside selon la revendication 1, **caractérisé en ce que** le nucléoside pentopyranosyle est un nucléoside ribo-, arabino-, lyxo- et/ou xylo-pyranosyle.

3. Lieur nucléoside selon l'une des revendications 1 ou 2, **caractérisé en ce que** le fragment pentopyranosyle du nucléoside pentopyranosyle est configuré en D ou L.

4. Lieur nucléoside selon l'une des revendications 1 à 3, **caractérisé en ce que** $R^2$, $R^3$, $R^4$, $R^{2'}$, $R^{3'}$ et/ou $R^{4'}$ représentent un reste 2-phtalimidoéthyle.

5. Procédé pour la préparation d'un lieur de formule (III), où $R^{4'}$ représente $(C_nH_{2n})NR^{10'}R^{11'}$ et $R^{10'}R^{11'}$ est lié par l'intermédiaire d'un reste de formule (V) ayant la signification indiquée à la revendication 1, **caractérisé en ce que**

(a) on fait réagir un composé de formule (III), où $R^{4'}$ représente $(C_nH_{2n})SO_{c3}$ ou $(C_nH_{2n})$ Hal, où n possède la signification donnée ci-dessus, $S_{c3}$ est un groupe protecteur et Hal représente un atome de chlore ou de brome, sur un azoture,
(b) on réduit le produit réactionnel de (a),
(c) on fait réagir le produit réactionnel de (b) sur un phtalimide correspondant,
(d) on fait réagir le produit réactionnel de (c) sur un pentose protégé correspondant,
(e) éventuellement on sépare la groupe protecteur.

6. Procédé pour la préparation d'un lieur selon la formule (I), où X et Y, indépendamment l'un de l'autre, identiques ou différents, chacun représente =C ($R^{16}$) -, où $R^{16}$ représente H ou $C_nH_{2n}$ et Z représente =C ($R^{16}$) -, où $R^{16}$ représente $(C_nH_{2n})$ $NR^{10}R^{11}$ ayant la signification donnée à la revendication 1,
**caractérisé en ce que**,

(a) on fait réagir l'indoline correspondante sur un pentose en nucléoside-triol,
(b) on protège les groupes hydroxyle du fragment pentose du produit de (a),
(c) on oxyde le produit de (b), et
(d) éventuellement on dissocie les groupes protecteurs d'hydroxyle du fragment pentose du produit de (c).

7. Procédé selon l'une des revendications 5 ou 6, **caractérisé en ce que** le nucléoside pentopyranosyle protégé en 4' est phosphitylé ou lié à une phase solide dans une étape ultérieure.

8. Procédé pour la préparation d'un acide nucléique, **caractérisé en ce que**,

(a) dans une première étape, un nucléoside protégé ou un lieur protégé selon l'une des revendications 1 à 4 est lié à une phase solide et
(b) dans une deuxième étape, le nucléoside protégé en 3',4', lié à une phase solide selon l'étape (a), est prolongé d'un nucléoside phosphitylé protégé en 3',4', ensuite il est oxydé, et
(c) l'étape (b) est répétée en utilisant des nucléosides ou lieurs nucléosides identiques ou différents jusqu'à l'obtention de l'acide nucléique voulu, l'acide nucléique renferme au moins un lieur nucléoside précité.

9. Procédé selon la revendication 8, **caractérisé en ce qu'**on incorpore à l'étape (a) et/ou l'étape (b) des nucléosides pentofuranosyle également au côté des nucléosides pentopyranosyle.

10. Procédé selon la revendication 8 ou 9 **caractérisé en ce que**, dans une étape ultérieure (d), le groupe protecteur et l'acide nucléique formé sont dissociés de la phase solide.

11. Procédé selon la revendication 10, **caractérisé en ce que** la dissociation est effectuée par hydrazinolyse.

12. Procédé selon l'une quelconque des revendications 1 à 5 **caractérisé en ce qu'**on incorpore, dans une étape ultérieure, un lieur allyloxy de formule

$$\mathbf{S_{c3}NH(C_nH_{2n})CH(OPS_{c5}S_{c6})C_nH_{2n}S_{c7}} \quad \mathbf{(VI)}$$

dans laquelle Sc4 et Sc7, indépendamment l'un de l'autre, identiques ou différents, chacun représente un groupe protecteur,
Sc5 et Sc6, indépendamment l'un de l'autre, identiques ou différents, chacun représente un groupe allyloxy et/ou diisopropylamino et n possède la signification donnée à la revendication 1.

13. Acide nucléique renfermant au moins un lieur nucléoside selon l'une des revendications 1 à 4 et au moins un lieur allyloxy selon la revendication 12.

14. Conjugué renfermant un lieur nucléoside selon l'une des revendication 1 à 4 et/ou un acide nucléique selon la revendication 13 et une biomolécule.

15. Conjugué selon la revendication 14 **caractérisé en ce que** la biomolécule est un peptide, un peptoïde, une protéine,

un composant cellulaire, un composant filamentaire ou un acide nucléique ou un dérivé de celui-ci.

**16.** Substrat **caractérisé en ce qu'**il porte immobilisé au moins un lieur nucléoside selon l'une des revendications 1 à 4, au moins un acide nucléique selon la revendication 13 et/ou au moins un conjugué selon la revendication 14 ou 15.

**17.** Produit diagnostique renfermant un lieur nucléoside selon l'une des revendications 1 à 4, un acide nucléique selon la revendication 13 et/ou au moins un conjugué selon la revendication 14 ou 15.

**18.** Utilisation d'un lieur nucléoside selon l'une des revendications 1 à 4, d'un acide nucléique selon la revendication 13, d'un conjugué selon la revendication 14 ou 15 et/ou d'un substrat selon la revendication 14 ou 15 et/ou d'un support selon la revendication 16, pour la préparation d'un médicament, d'un produit diagnostique et/ou composant électronique.

**19.** Utilisation d'un lieur nucléoside selon l'une des revendications 1 à 4, d'un acide nucléique selon la revendication 13, d'un conjugué selon la revendication 14 ou 15 et/ou d'un substrat selon la revendication 16, dans un système d'appariement et/ou de test.

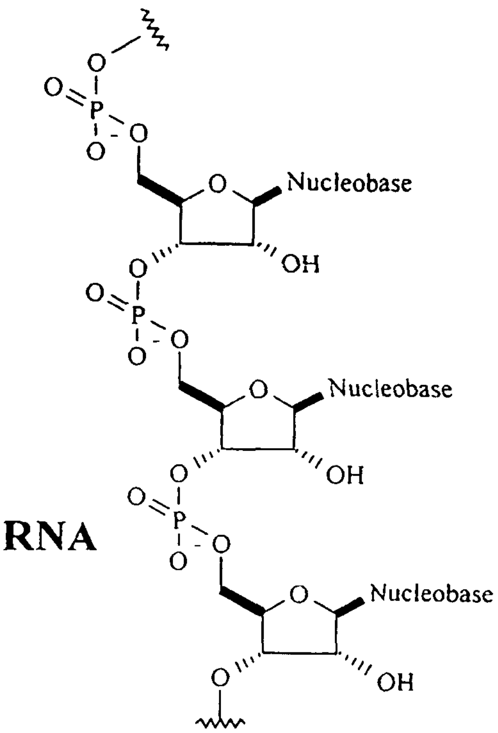

RNA

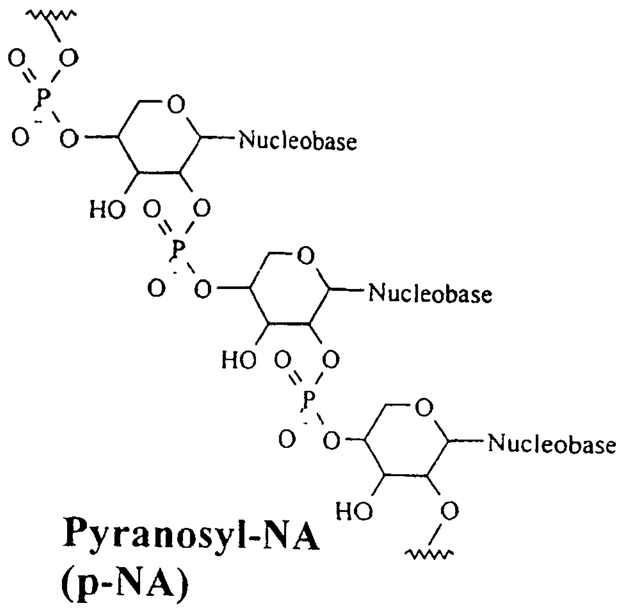

Pyranosyl-NA
(p-NA)

Fig. 1.

# Fig. 2A

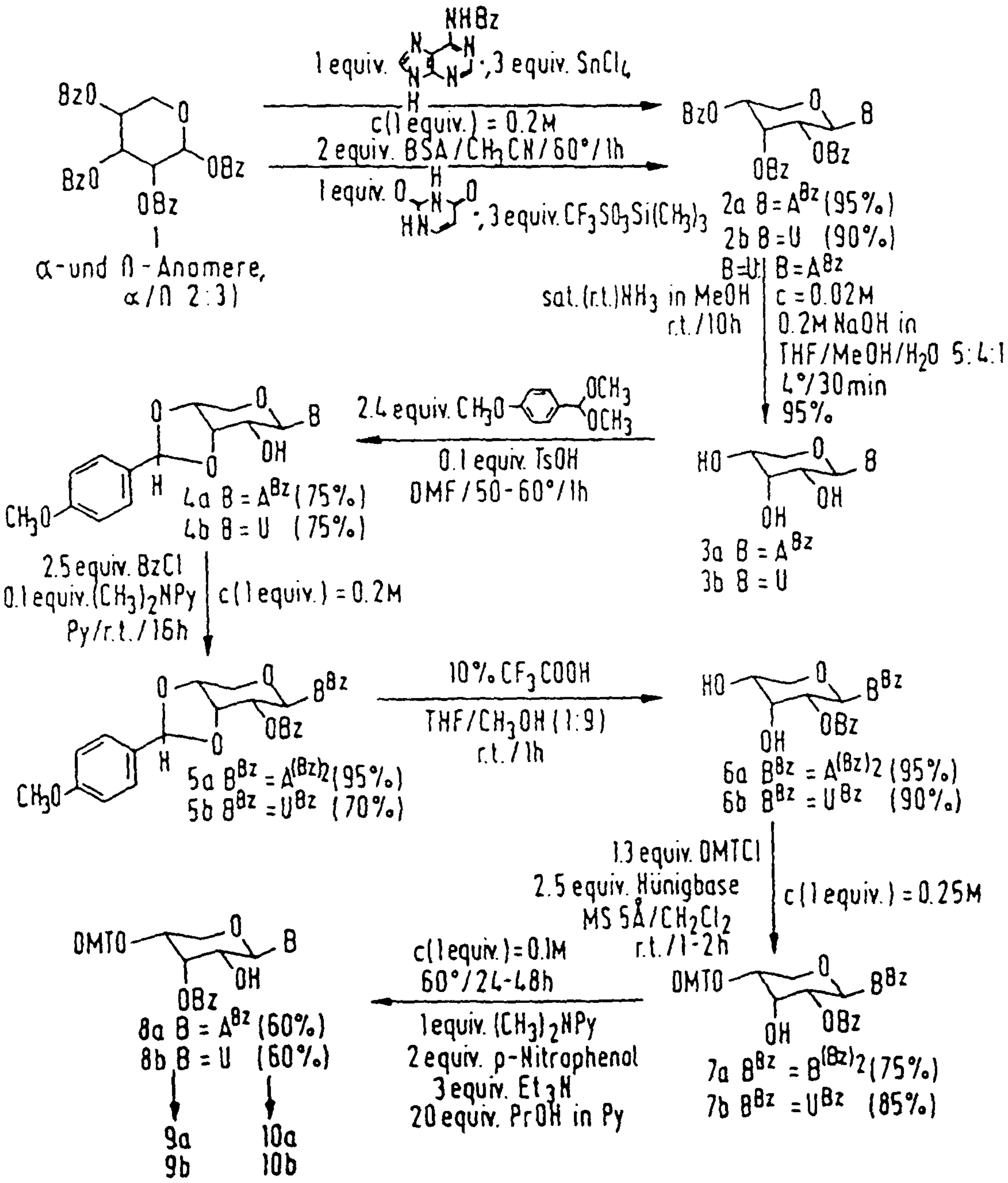
1 equiv. ; 3 equiv. $SnCl_4$
c(1 equiv.) = 0.2M
2 equiv. BSA/$CH_3CN$/60°/1h
1 equiv. ; 3 equiv. $CF_3SO_3Si(CH_3)_3$
1
α- und β-Anomere,
α/β 2:3)
2a B = $A^{Bz}$ (95%)
2b B = U (90%)
B=U
sat.(r.t.) $NH_3$ in MeOH
r.t./10h
B = $A^{Bz}$
c = 0.02M
0.2M NaOH in
THF/MeOH/$H_2O$ 5:4:1
4°/30min
95%
3a B = $A^{Bz}$
3b B = U
2.4 equiv.
0.1 equiv. TsOH
DMF/50-60°/1h
4a B = $A^{Bz}$ (75%)
4b B = U (75%)
2.5 equiv. BzCl
0.1 equiv. $(CH_3)_2NPy$
Py/r.t./16h
c(1 equiv.) = 0.2M
5a $B^{Bz}$ = $A^{(Bz)2}$ (95%)
5b $B^{Bz}$ = $U^{Bz}$ (70%)
10% $CF_3COOH$
THF/$CH_3OH$ (1:9)
r.t./1h
6a $B^{Bz}$ = $A^{(Bz)2}$ (95%)
6b $B^{Bz}$ = $U^{Bz}$ (90%)
1.3 equiv. DMTCl
2.5 equiv. Hünigbase
MS 5Å/$CH_2Cl_2$
r.t./1-2h
c(1 equiv.) = 0.25M
7a $B^{Bz}$ = $B^{(Bz)2}$ (75%)
7b $B^{Bz}$ = $U^{Bz}$ (85%)
c(1 equiv.) = 0.1M
60°/24-48h
1 equiv. $(CH_3)_2NPy$
2 equiv. p-Nitrophenol
3 equiv. $Et_3N$
20 equiv. PrOH in Py
8a B = $A^{Bz}$ (60%)
8b B = U (60%)
9a
9b
10a
10b

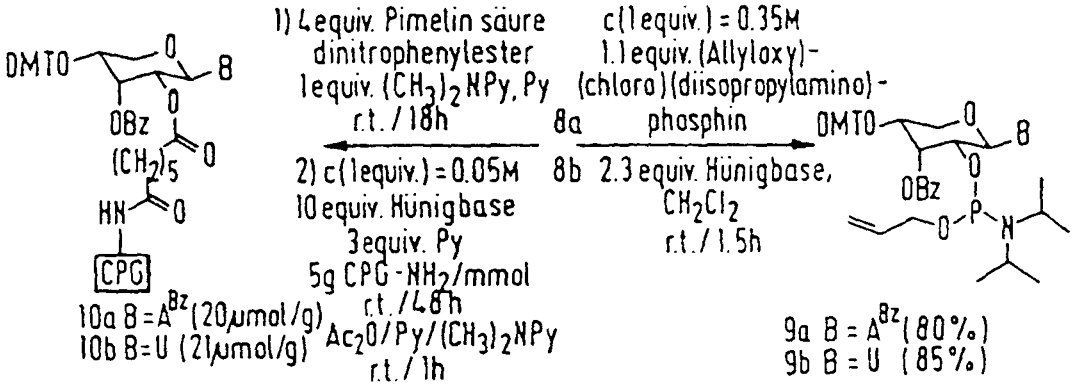


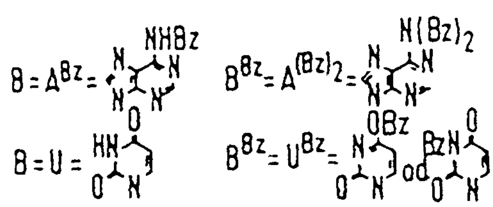


Bz = 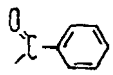

$(CH_3)_2NPy$ = 4-(Dimethylamino)pyridin

DMT = 4,4'-Dimethoxytrityl

BSA = Bis(trimethylsilyl) acetamide

CPG = 'long-chain-alkylamino-controlled pore glass' (Sigma)

Fig. 2B

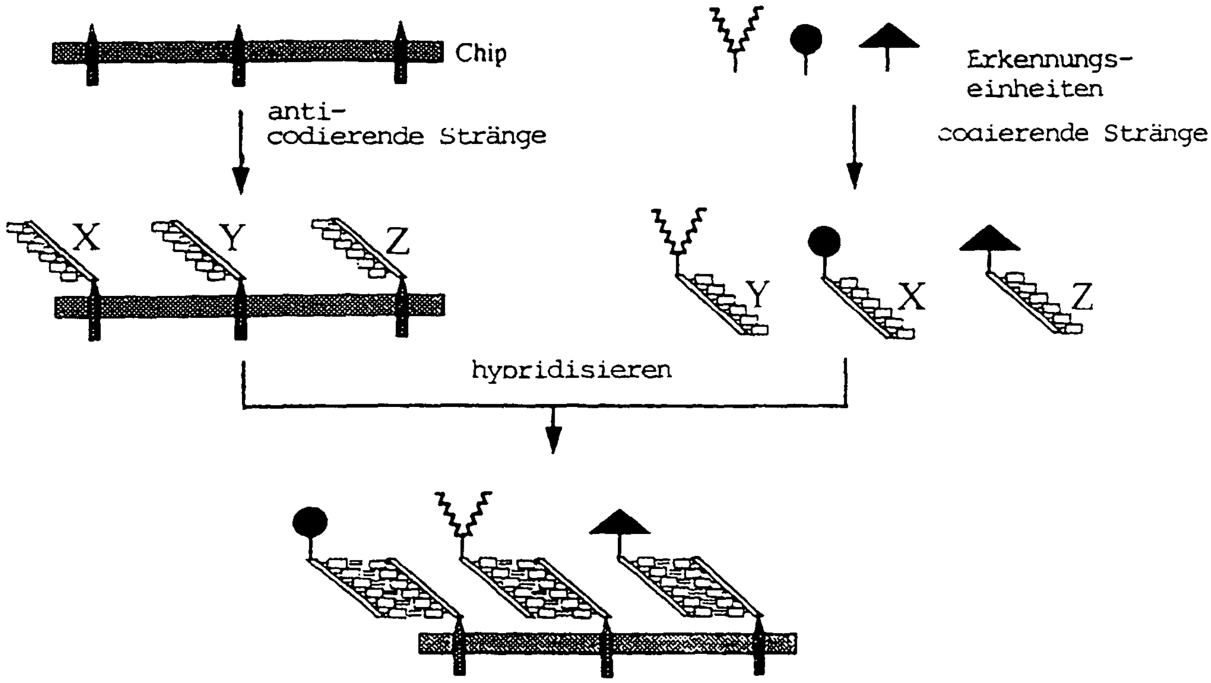
Chip
anti-
codierende Stränge
Erkennungs-
einheiten
codierende Stränge
X
Y
Z
Y
X
Z
hybridisieren

Fig. 3